# EUROPEAN PATENT SPECIFICATION

(11) **EP 1 483 375 B1**
(45) Date of publication and mention of the grant of the patent: **21.01.2009**
(21) Application number: 03708709.5
(22) Date of filing: 08.03.2003
(51) Int. Cl.: C12N 7/01, C12N 15/86

(54) **A METHOD FOR PRODUCING RECOMBINANT ADENOVIRUS VIRUSES USING SITE-SPECIFIC RECOMBINATION**
VERFAHREN ZUR HERSTELLUNG REKOMBINANTER ADENOVIRALER VIREN MITTELS STELLENSPEZIFISCHER REKOMBINATION
PROCEDE DE PRODUCTION DE VIRUS RECOMBINANTS ADENOVIRAUX A L'AIDE DE LA RECOMBINAISON A UN SITE SPECIFIQUE

(30) Priority: 09.03.2002 KR 2002012633
(43) Date of publication of application: 08.12.2004
(73) Proprietor: NEWGEX Inc., Kwanak-gu Seoul (KR)
(72) Inventor: SON, Ho-Sun, Gwanak-gu, Seoul 151-812 (KR); JUNG, Neon-C, Seoul 151-770 (KR); SEEN, Dong-Seung, Seoul 151-812 (KR); KIM, Kyung-Jin, Seoul 120-757 (KR); CHO, Sung-Eun, Yongin Gyeonggi-do 449-843 (KR); CHOI, Eun-Wook, Gwanak-gu Seoul 151-050 (KR)
(74) Representative: W.P. Thompson & Co.
(86) International application number: PCT/KR2003/000453
(87) International publication number: WO 2003/076605

(56) References cited:
- US-A- 6 143 557
- WALHOUT A J M ET AL: "GATEWAY RECOMBINATIONAL CLONING: APPLICATION TO THE CLONING OF LARGE NUMBERS OF OPEN READING FRAMES OR ORFEOMES" METHODS IN ENZYMOLOGY, ACADEMIC PRESS INC, SAN DIEGO, CA, US, vol. 328, 2000, pages 575-592, XP001056139 ISSN: 0076-6879
- HARTLEY ET AL.: 'DNA cloning using in vitro site-specific recombination' GENOME RESEARCH vol. 10, no. 11, November 2000, pages 1788 - 1795, XP002187669
- HARDY S. ET AL.: 'CONSTRUCTION OF ADENOVIRUS VECTORS THROUGH CRE-LOX RECOMBINATION' J. VIRO. vol. 71, no. 3, March 1997, pages 1842 - 1849, XP000670223
- PARKS R.J. ET AL.: 'HELPER-DEPENDENT SYSTEM FOR ADENOVIRUS VECTOR PRODUCTION HELPS DEFINE A LOWER LIMIT FOR EFFICIENT DNA PACKAGING' J. VIRO. vol. 71, no. 4, April 1997, pages 3293 - 3298, XP000886850
- LOGVINOFF ET AL.: 'Genetic Engineering of Herpes Simplex Virus and Vector Genomes Carrying loxP Sites in Cells Expressing Cre Recombinase' VIROLOGY vol. 267, 2000, pages 102 - 110, XP000907694

## Description

### TECHNICAL FIELD

This invention relates to a method for producing recombinant viruses and vectors for the same.

### BACKGROUND

A recombinant viral vector means a genetically engineered vector, which is used in, for example, the preparation of a vaccine; analysis of function of a gene, a group of genes, or a genomic domain; production of proteins; and gene therapy. Genomic materials, which could be integrated into a viral vector, include any genomic materials such as a gene, cDNA, genomic DNA, DNA sequences encoding peptides or proteins, RNA, anti-sense RNA, siRNA, DNA for si RNA, promoter, enhancer etc. A virus, which is used in the preparation of a vector, includes adenovirus, adeno-associated virus (AAV), retrovirus, Herpes virus and Vacxnia virus etc.

Retrovirus is known as one of the most widely used viruses in gene therapy [see Clive Patience et al. J Virol, 72:2671-2676, 1998, Marshall, Science, 269: 1050-1055,1995]. Retroviral genome has two LTRs, capsid sequences and 3 (three) coding regions (gag, pol and env), and the method of its preparation and its use *in vitro* and *in vivo* has been already disclosed [WO9908692A1 and EP 453242]. The clinical application of retroviral vector, however, has the following disadvantages as follows: it forms a replication-competent retrovirus (RCR) (Sharon K. Powell et al. J.Virol., 73:8813-8816, 1999); it has relatively lower gene expression level, and the level decreases continuously *in vivo*; and the titer of virus is low. Furthermore, the retrovirus cannot transfer a gene to a non-dividing cell [Jaffe, E.M. Cancer Res., 53:2221-2226, 1993; Bender, M.A. et al. J. Virol., 61:1639-1646, 1987].

Adenovirus has a linear genome (sized about 36 kb) and includes replication origin and capsid signals near to the left inverse terminal region (ITR) (103 bp) [Shenk, Adenoviridae: The Viruses and Their Replication. In: Fields BN, Knipe DM Howley PM, ed. Virology. Philadelphia: Raven publishers, 1996: 2111-2148]. Usually, adenoviral vectors have been made by deleting E1 site of the viral genome, which is essential for the viral replication. The viruses, which include foreign genomic materials substituting E1 site, are transferred to the packaging cells that provide E1 proteins. Thereby, the viral replication occurs only in the packaging cells. Representative packaging cells include, for example, HEK293 cell, 911 cell, and PER. C6 cell, etc. [Hitt MM et al., Advances in Pharmacology, vol. 40, 137-206 (1997), Wang Y. and Huang S., Drug]. Adenoviral vector has advantages such as safty, affinity to various cells, possibility of infecting dividing cells and higher titer (10¹¹ pfu/ml). Thus, adenovirus has been used in the preparation of a vector expressing heterogeneous genes.

Adeno-associated virus (sized about 4700 bp) has ITRs (sized about 145 bp) as replication origins at each terminus. It can be integrated into the genomic DNAs of various types of host cells safely and specifically.

In order to use a recombinant virus as a vector, it should be modified not to replicate themselves in the infected cells. Therefore, defective viruses, which have deletions of some essential regions of genome for viral replication, are usually employed in the preparation of the recombinant viral vectors. In case of a retrovirus, for example, gag, pol and/or env genes are deleted, and the regions are replaced with desired genomic materials [Bender et al., J. Virol. 61 (1987) 1639]. Regarding an adenovirus, E1, E2 and/or E4 sites are deleted for the preparation of a viral vector [Levrero et al., Gene 101 (1991) 195; Gosh-Choudhury et al., Gene 50 (1986) 161, Van der Vliet et al., (1975)]. On the other hand, a pseudo-virus vector, which consists of only the essential regions (such as, ITR and capsid sequences) of the replication, has been used also for obtaining recombinant viruses (see WO95/02697).

More specifically, conventional methods for the preparation of adenoviruses could be grouped into 3 categories as follows: In the first group, homologous recombination between carrier vectors and adenoviral genomic DNAs is provided, wherein the vectors carry desired foreign genomic materials [Hitt MM et al., Advances in Pharmacology, vol. 40, 137-206 (1997)]. In this method, desired foreign genomic materials are cloned into carrier vectors and then introduced to the packaging cells together with adenoviral vector genomic DNAs in order to induce homologous recombination. A carrier vector usually includes a cassette, such as a promoter, restriction enzyme target sites for gene cloning and poly(A) attachment signals, for the expression of foreign genomic materials. Furthermore, the carrier vector has nucleic acid sequences inducing homologous recombination. In general, a defective adenoviral genome, which lacks LITR, packaging signals, or E1 site, is used in this method. On the other hand, Frank Graham et al. designed a plasmid type vector, which can replicate in E. coli, in order to resolve the problem of the said method of requiring a further step for selecting defective viral genomes from viruses [Bett AJ et al., Proc. Natl. Acad. Sci USA,, vol 91, 8802-8806 (1994)]. The Graham's method, however, also has to use the homologous recombination between carrier vectors and defective adenoviral genomic DNAs, and the lower rate of homologous recombination in packaging cells also made this method inefficient.

The second group includes direct cloning of desired foreign genomic materials to a certain region of adenoviral genome using restriction enzyme and ligase. This method resolved the difficulties caused by the structural complexity of the adenoviral genome (sized about 30 kb). In this method, expression cassette for the desired gene is, firstly, cloned to a carrier vector, which in turn is cloned again into the viral genome using specific restriction enzyme sites within both of the cassette and the viral genome. Next, the cloned cassette and the viral genome are introduced into E. coli, and the desired clones are selected. Afterward, the selected clones are introduced to the packaging cells. Then, the recombinant viruses could be obtained from the packaging cells [Mizuguchi H. and Kay MA., Human Gene Therapy, vol. 10, 2013-2017 (1999)]. In another method of this group, viral genomic DNAs having pTP (preterminal protein) at the region of ITR terminus are extracted from viruses, not from E. coli, in order to increase the productivity of desired recombinant viruses, which in turn are transferred to cell lines after being ligated with the desired genome expression cassette [Okada T. et al., Nucleic Acids Research, vol. 26, 1947-1950 (1998)].

In the third group, a method employs E. coli, which has relatively higher efficiency of homologous recombination, in order to improve the lower recombination rate in the packaging cells [Chartier C. et al., Journal of Virology, vol. 70, 4805-4810 (1996), He T. et al., Proc. Natl. Acad. Sci USA, Vol 95, 2509-2514 (1998)]. According to this method, although the desired genomic materials should be also cloned into carrier vectors, it is possible to obtain relatively higher rate of homologous recombination in the particular E. coli by infecting the cell together with plasmid-type adenoviral genome. Thereby, the desired recombinant viruses could be obtained with higher productivity in comparison to the methods mentioned above. In this method, however, the desired genomic materials should be cloned to carrier vectors. And, a particular E. coli cell line (for example, BJ5183) should be used. Furthermore, the recombinant DNAs expressed in E. coli should be screened, and the screened recombinant DNAs should be transferred to another E. coli cell line (for example, DH5 α) due to the unstableness of the plasmid in the E coli used for the recombination. Thus, this method is considered as being very complex and not effective.

A review of GATEWAY ^{™} recombinatorial cloning C. elegans ORFs into a versatile vector and from that vector into two different expression vectors used for two-hybrid analysis has been described. [Walhout AJM. et al, methods in Enzymology, vol. 328, 575-592 (2000)].

US 6143557 discloses recombinational cloning by the use of nucleic acids, vectors and methods, *in vitro* and in vivo, for moving or exchanging segments of DNA molecules using engineered recombination sites and recombination proteins to provide chimeric DNA molecules that have the desired characteristic(s) and/or DNA segment(s).

As disclosed above, the conventional methods for the preparation of recombinant viruses have disadvantages as follows: i) Desired genomic materials should be cloned to carrier vectors; ii) Since the gene cloning of the desired genomic materials should be carried out in a cell, it requires complex and inefficient cloning procedures; iii) The desired recombinant viruses should be selected from host cells before the mass production. Therefore, according to those methods, it takes much time to obtain recombinant viruses, while the productivity is relatively low. Especially, the disadvantages of ii) and iii) have been regarded as the most important problems to be overcome in the preparation of recombinant viruses, thus there have been continuous efforts to resolve these problems so far. On the other hand, according to the development of applications of recombinant viruses, the requirements for more efficient and rapid method for preparing recombinant viruses are increasing.

Thus, we have carried out studies to produce recombinant viruses having desired genes, and we have completed this invention by providing recombinant viral vectors using *in vitro* site-specific recombination.

### DETAILED DESCRIPTIONS OF THE INVENTION

It is important to understand the present invention to note that all technical and scientific terms used herein, unless otherwise defined, are intended to have the same meaning as commonly understood by one of ordinary skill in the art. The techniques used herein are also those that are known to one of ordinary skill in the art, unless stated otherwise.

Reference to particular buffers, media, reagents, cells, culture conditions and the like, or to some subclass of same, is not intended to be limiting, but should be read to include all such related materials that one of ordinary skill in the art would recognize as being of interest or value in the particular context in which that discussion is presented. For example, it is often possible to substitute one buffer system or culture medium for another, such that a different but known way is used to achieve the same goals as those to which the use of suggested method, material or composition is directed.

It is an object of this invention to provide more rapid and more efficient method for producing recombinant viruses.

In order to accomplish this invention, the present invention employs recombinant viral vectors, which are prepared by *in vitro* site-specific recombination. More preferably, the viruses used in this invention have linear genomic DNAs.

Therefore, it is another object of the present invention to provide recombinant viral vectors using in vitro site-specific recombination.

In accordance with the present invention, there is provided a method for preparing linear recombinant-adenoviral vectors, wherein desired DNAs are inserted into adenoviral genomic DNAs by *in vitro* site-specific recombination, characterized in that the method comprises:
i) providing adenoviral genomic DNA segments containing *att* site-specific recombination sites and
   base sequences essential to viral replication and packaging,
ii) providing carrier vectors comprising at least one desired DNAs and *att* site-specific recombination sites; and
iii) reacting the DNA segments of i) and the carrier vector of ii) *in vitro.*

In accordance with another aspect of the present invention, there is provided a method for preparing recombinant adenoviruses having linear genomic DNA, characterized in that the method comprising the steps of:
i) providing adenoviral genomic DNA segments containing *att* site-specific recombination sites and base sequences essential to viral replication and packaging;
ii) providing carrier vectors containing at least one desired DNAs and *att* site-specific recombination sequences;
iii) reacting the genomic DNA segments of i) and the carrier vectors of ii) *in vitro* using site-specific recombinase to produce mixture comprising expressive linear recombinant adenoviral vectors; and
iv) transfecting the mixture of iii) to host cells.

In the present invention, a vector comprising viral genomic DNAs is cleaved to two or more segments before being applied to *in vitro* site-specific recombination, in which both of the segments are essential for the viral replication. A DNA fragment including desired genomic materials is inserted between the two genomic DNA segments specifically. Thus, since un-bound viral genomic DNA segments cannot be expressed by themselves, only the viruses having desired DNA fragment shall be expressed. Therefore, one can obtain desired recombinant viruses from host cells directly without further procedures like selecting the desired recombinant viruses from intermediate host cells.

Referring to the site-specific recombination, it is an event occurring naturally in various living organisms. Enzymes for the site-specific recombination contain not only endonuclease activity but also ligase activity, so they recognize a certain part of DNA sequences and replace it with any other corresponding DNA sequences [Yang W. and Mizuuchi K., Structure, 1997, Vol. 5, 1401-1406(9)]. Int/att system from bacterio λ phage, Cre/LoxP system from P1 bacterio phage and FLP-FRT system from yeast are well developed site-specific recombination systems.

Site-specific recombination system has been used in i) expression control of desired genomic materials cloned into recombinant viruses, ii) excising of helper-virus when preparing helper-viral dependent viruses, iii) increasing the efficiency of homologous recombination in the packaging cells, or iv) inducing site-specific recombination between carrier vectors and viral genome in the packaging cells to prepare recombinant viruses [Philip NG et al., Biotechniques, vol. 29. 524-528 (2000), Philip NG et al., Human Gene therapy, vol. 10., 2667-2672 (1999), Hardy S et al., Journal of Viology, vol. 71., 1842-1849 (1997), Parks RJ et al., Proc. Natl. Acad. Sci USA,, vol 93., 13565-13570 (1996)]. However, the said applications of the site-specific recombination disclosed in the prior arts were merely indirect and supplementary uses in vivo or in a cell line, not *in vitro.*

Thus, there have been no applications of *in vitro* site-specific recombination for specific insertion of desired genomic materials into linear viral genomic DNAs in order to obtain recombinant viral vectors. In particular, there have been no applications of using viral genomic DNAs, which are separated into two segments.

Therefore, the present invention is characterized by employing *in vitro* site-specific recombination in the preparation of recombinant viral vectors. It has unexpected advantages in comparison to the prior arts as follows: i) it is possible to obtain recombinant viral vectors more easily and more rapidly by employing *in vitro* site-specific recombinant; ii) it does not require any further procedure, such as plaque assay, for selecting desired recombinant viruses from intermediate host cells by digesting viral genomic DNAs to two segments before applying them to site-specific recombination; and iii) it is possible to apply the reaction mixture including recombinant viral vectors to host cells without further processes.

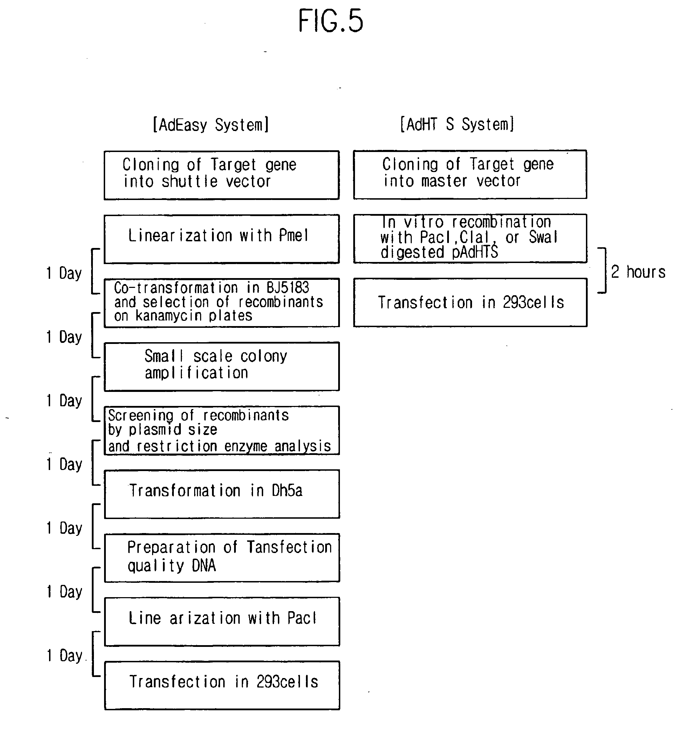

Furthermore, according to this invention, homologous recombination in the packaging cells is not required. Nor, particular cell lines for inducing homologous recombination in E. coli are required. FIG. 5 shows the results of comparisons between the method of this invention for the preparation of adenovirus (AdHT System) and the method of the prior art (AdEasy system) in view of processes and time-period. From the results, it is clear that the desired recombinant viruses can be obtained even more rapidly by using the method of this invention.

Furthermore, according to the present invention, since numerous kinds of desired genomic materials are integrated into the sites of viral genomic DNAs accurately *in vitro,* various genomic materials can be expressed from the same kind of virues in multi-well (e.g. 96-well and 384-well) at the same time. Likewise, since numerous kinds of viral genomes from various types of viruses [such as, adenovirus, retrovirus, adeno-associated virus, bacteriophage, herpes simplex virus, CMV (cytomegalovirus), EBV (Epstein-Barr virus) or VZV (Varicella-zoster virus), etc] are employed, the same kind of genomic material can be expressed from various types of viruses by using an universal common vector (pMaster Vector) containing the desired genomic material.

Accordingly, the present invention also provides significantly useful tools for studying functional genomics of which demands have been increasing greatly, since the completion of genome project [Wang Y. and Huang S., Drug Discovery Today, vol. 5, 10-16 (2000)].

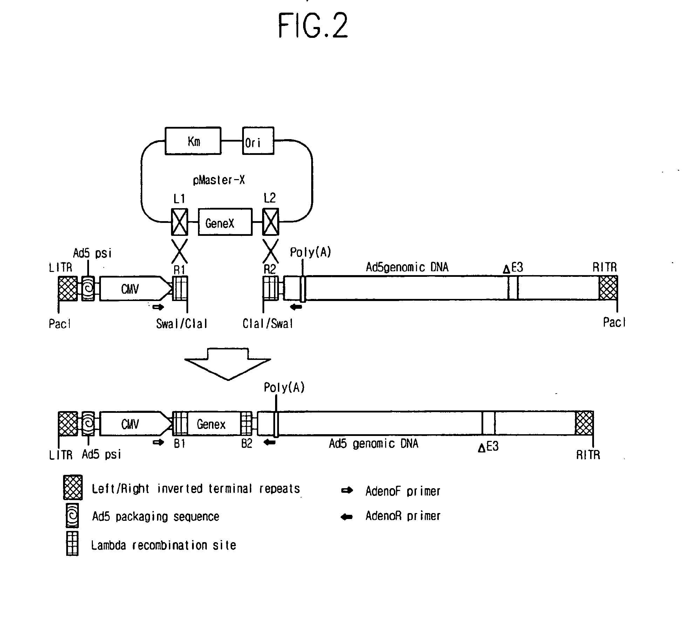

In one embodiment, this invention provides recombinant adenoviruses of which genomic DNAs are in linear form. As depicted in FIG. 2, genomic DNAs of viruses are firstly cleaved to two segments, wherein the segments respectively are essential for the viral replication and packaging. Then, using the cleaved segments as templates, the desired genomic materials containing site-specific recombination sites are integrated through site-specific recombination. More specifically, E1 site deleted adenovirus is digested to two segments, and recombination sites are introduced into each of the segments in order to induce *in vitro* site-specific recombination. Other corresponding site-specific recombination sites are introduced into the desired genomic materials.

Referring to FIG. 2, the left segment of adenoviral genome includes ITR and packaging signals, and the right segment includes other genes essential for viral replication. Recombination and viral packaging, however, cannot occur solely with only one of the two cleaved segments (since the right segment lacks signals for particle formation and the left segment is too short to form a viral particle). Thus, only the viral genome containing the desired genomic materials, which are integrated by site-specific recombination, can be used as a template for replication in a packaging cell.

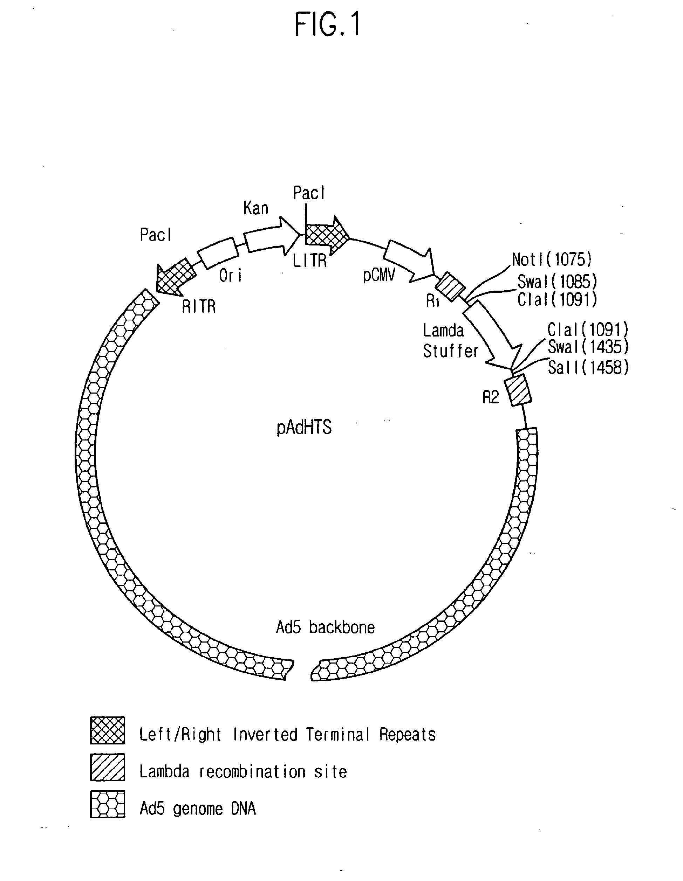

On the other hand, methods disclosed below can be used for obtaining two segments of viral genomic DNAs. In the first method, viral genomic DNAs are cleaved to two segments with restriction enzyme, wherein each of the segments is essential for viral packaging and has a site-specific recombination sites (see FIG. 2). More specifically, one or more restriction enzyme recognition sites are firstly cloned to viral genomic DNAs (see FIG. 1), and then two or more viral DNA segments can be obtained by restriction enzyme treatment. The viral genomic DNAs including restriction enzyme recognition sites could be obtained as a plasmid from E. coli, or be obtained directly from viruses. The obtained plasmid (pAdHTS) has a construct as depicted in FIG. 1. Next, pAdHTS (SEQ. ID No: 1) is cleaved to three segments by restriction enzyme treatment. Except for the segment not related to site-specific recombination and viral replication, other two segments are employed in the preparation of recombinant viral vectors (see FIG. 2).

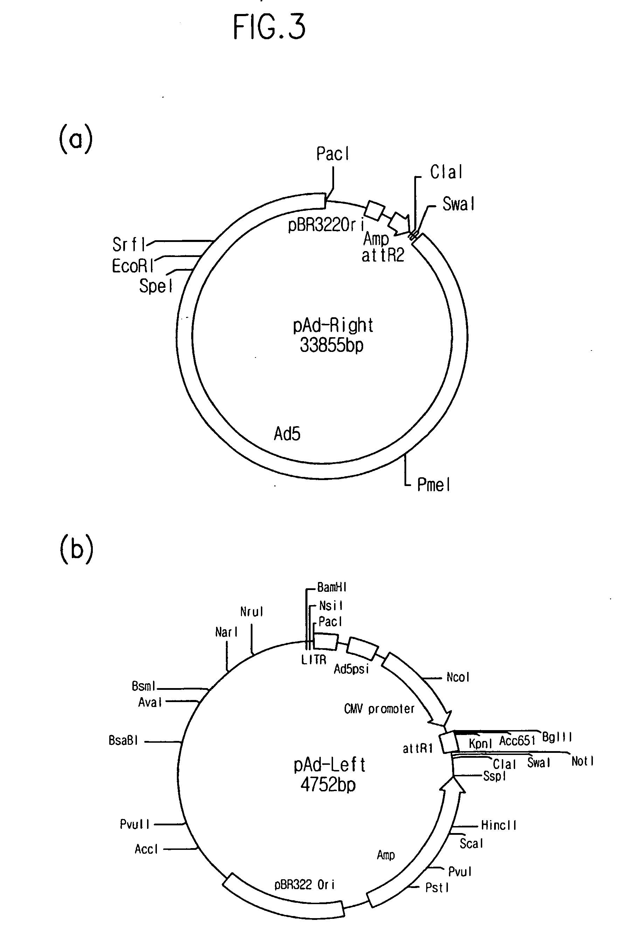

In the second method, parent viral formation can be prevented. As disclosed in Example 3, the left segment and the right segment of viral DNAs are generated as plasmids respectively, which in turn are made to linear forms, wherein the two segments have site-specific recombination sites, which are different from each other. Referring to FIG. 3, left-segment has ITR and packaging signals, and the right-segment has nucleic acids essential for the viral replication. Each of the segments has replication origins (e.g. pBR322 origin or pUC origin etc.) for the replication of DNAs in E. coli, and at least one selection marker (e.g. Ampr, Kanr, Cmr, etc) to permit detection and/or selection. These segments can be provided from other plasmids, respectively.

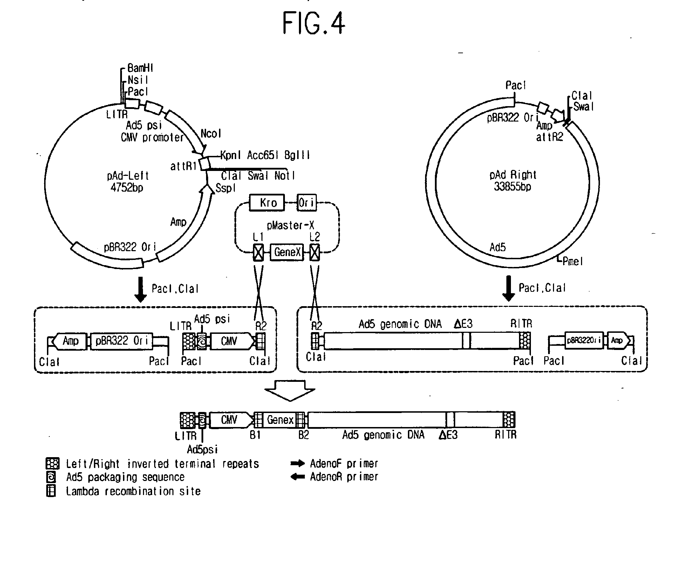

The viral genomic DNA segments in this invention can be in linear or in circular form. Preferably, they are in linear form. For example, if linear DNA segments generated by restriction enzyme treatment are integrated into pMaster vectors *in vitro,* linear formed recombinant viral genome could be obtained as depicted in FIG. 4. Thus, in case of circular plasmid DNAs, they are firstly treated with restriction enzyme, such as *PacI*, to form linear DNA segments and consequently to obtain linear viral genome (see FIG. 4), and then are introduced into the packaging cells to induce viral replication.

In one embodiment, a promoter is inserted into at a site of viral genomic DNAs in order to induce the expression of desired genomic materials (see FIG. 1 and FIG. 2). The promoter, not operating in a cleaved DNA segment, induces the expression of genomic materials, when the materials are introduced by site-specific recombination. In FIG. 6, green fluorescence proteins (GFP) and Gus genes are not expressed in the group of cells that has not been subjected to site-specific recombination.

A promoter, which can be employed in the present invention, includes CMV promoter for continuous gene expression, RSV (Respiratory syncytial virus) promoter, SV (simian virus) promoter, a promoter system which induces gene expression only in specific environment, a promoter system containing ERE which operates in response to corresponding hormone, a promoter system operating in response to ecdysone, and a promoter system operating in response to tetracycline, etc. The promoter typically controls the expression, optionally with an operator sequence.

In another embodiment, attR sites within two viral genomic DNA segments react with attL sites from carrier vectors including foreign genomic materials, and form attB and attP sites. More specifically, when the two viral genomic DNA segments are treated with carrier vectors having desired genomic materials in the presence of λ integrase, IHF as a co-factor and Xis, attB sites are generated as main products. Vector DNA segments having attP sites are generated also as by-products in the reaction mixture. Numerous recombination systems from various organisms, for example, integrase/att system from bacteriophage λ, the Cre/loxP system from bacteriophage P1, and the FLP/FRT system from the Saccharomyces cerevisiae 2 µ circle plasmid, can be employed.

If the mixtures are transferred directly to suitable host cells, which are expected to express recombinant viruses, only the viral genomic DNAs containing attB1-[desired genomic materials]-attB2 can replicate and form viral particles. The viral DNA segments and the vector DNAs, which do not react with each other, and the vector DNA segments having attP sites do not form viral particles.

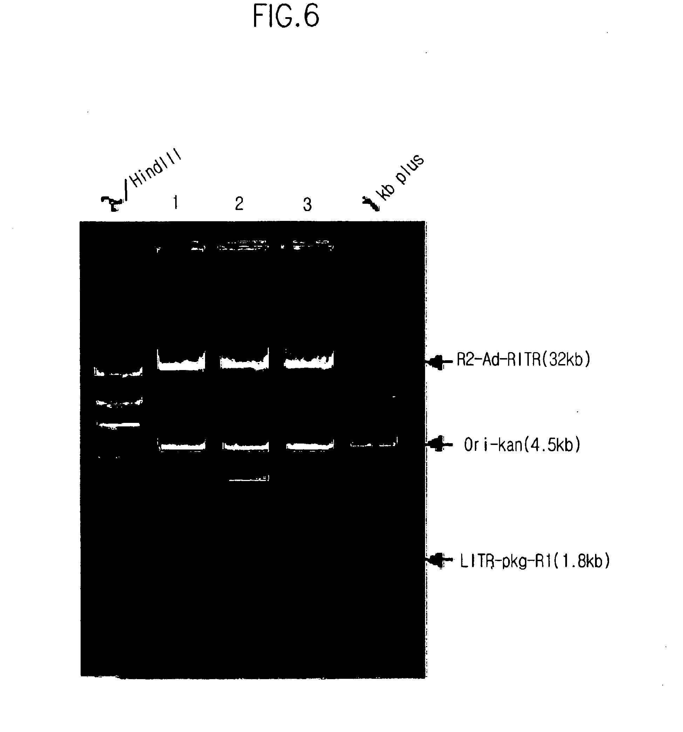

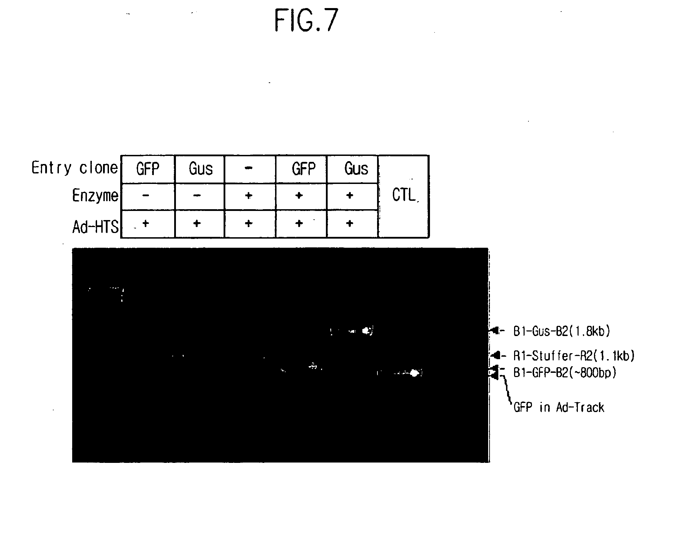

Referring to FIG. 6, the disappearance of left segments of viral genomic DNAs suggests that the DNA segments having attR sites are recombined. In FIG. 7, PCR products show that the desired genomic materials are inserted exactly into suitable sites of viral genome. PCR reaction is carried out for both of the samples collected before- and after-recombination reaction using primers (Adeno-F primer and Adeno-R primer) that are able to amplify certain regions of viral genome.
Adeno-F primer: 5'-GGTCTATATA AGCAGAGCTG-3' (SEQ. ID No.: 2)
Adeno-R primer: 5'-GTATGGCTGA TTATGATCAG-3' (SEQ. ID No.: 3)

The analysis of PCR products using DNA sequencing shows that viral genome and desired genomic materials are integrated at the target sites and generated [left segment of viral genome-attB1-desired genomic materials-attB2-right segment of viral genome] (see FIG. 2).

Viral replication and packaging are observed after infecting the packaging cells, such as 293 cells, with the reaction mixture including site-specific recombinant viral vectors. These results indicate that the *in vitro* mixture of this invention can be applied to host cells directly to produce recombinant viruses without further step of selection. That is, according to this invention, without employing intermediate host cells, such as E. coli, for selecting appropriate genomic DNAs, desired recombinant viruses can be produced rapidly.

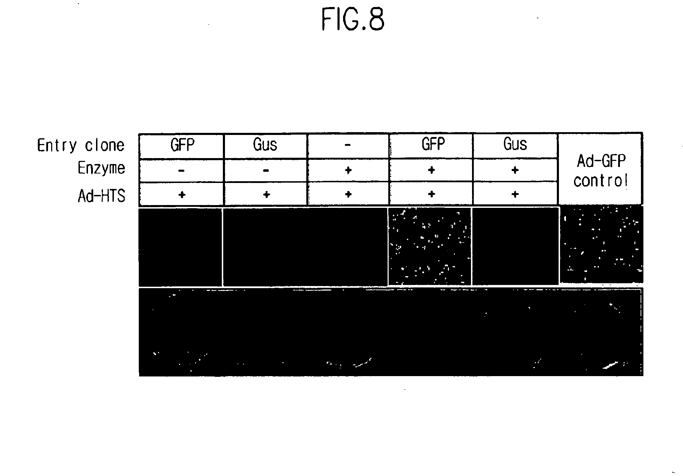

In FIG. 8, the results of site-specific recombination of AdHTP vectors and pMater clones are presented. The expression efficiency of GFP was still higher in the method of this invention compared to the method of the prior art (Ad-GFP control). The activity assays of Gus proteins indicated that the method of this invention did not have adverse-effects on the activities of the proteins expressed. Thus, the recombinant viruses produced in this invention are suitable for the expression of desired genomic materials from infected host cells.

### BRIEF DESCRIPTIONS OF FIGURES

FIG.1 depicts the construct of pAdHTS vector.
FIG.2 shows the process of site-specific recombination between pAdHTS vector and pMaster clone *in vitro.*
FIG.3 depicts the constructs of pAd-Left and pAd-Right.
FIG.4 shows the process of site-specific recombination of pAd-Left/pAd-Right and pMaster clone *in vitro.*
FIG.5 shows processes and time-period for the production of recombinant adenovirus using AdHTS in comparison to those of the methods of prior arts.
FIG.6 illustrates agarose gel eletrophoretic analysis showing the results of site-specific recombination of pAdHTS vector and pMaster clone *in vitro.*
FIG. 7 shows the results of PCR, which indicate site-specific recombination of pAdHTS vectors and pMaster clones.
FIG. 8 shows the results of site-specific recombination of AdHTS vectors and pMaster clones. The desired genomic materials in pMaster clones are transferred to adenoviral genome to express the desired proteins.

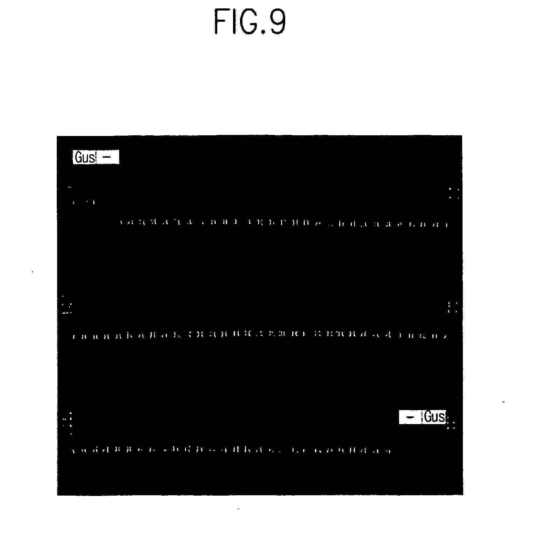

FIG. 9 shows the results of PCR indicating the site-specific recombination of AdHTS and pMaster-GFP in 96-well.

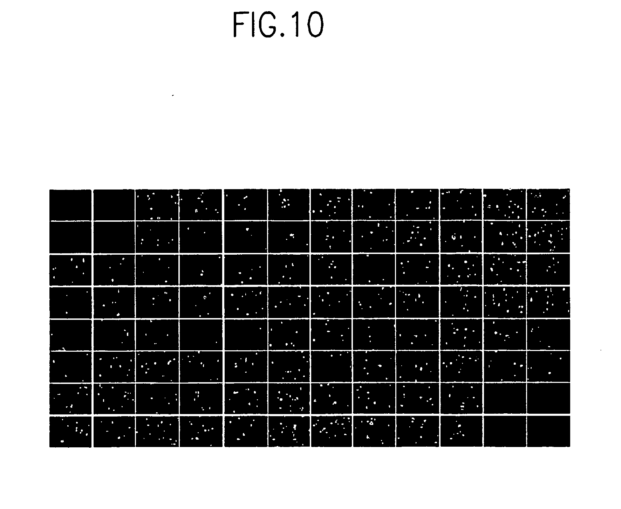

FIG.10 shows the results of site-specific recombination of AdHTS and pMaster-GFP in 96-well. GFP gene inserted into pMaster clone is transferred to adenoviral genome to express GFP

Preferred embodiments of this invention are described in the following examples. Other embodiments within the scope of the claims herein will be apparent to those skilled in the art from consideration of the specification or practice of the invention as disclosed herein. It is intended that the specification, together with the examples, be considered exemplary only, with the scope and the spirit of the invention being indicated by the claims which follow the examples. The examples herein are meant to exemplify the various aspects of carrying out the invention and not intended to limit the scope of the invention in any way. The examples do not include detailed descriptions of conventional methods employed, such as in the performance of genomic DNA isolation, PCR, and sequencing procedures. Such methods are well-known to those skilled in the art and are described in numerous publications. In addition, all the publications referred herein are integrated hereto as references.

### EXAMPLES

### EXAMPLE 1: Site-specific recombination using pAdHTS

pMaster vector having a desired genomic material flanked with attL sites was prepared (see FIG.2). Then, pAdHTS (SEQ. ID No.: 1) having viral genomic DNA flanked with attR sites was prepared using conventional recombination method (see FIG.1). The pAdHTS vector for this study was generated by deleting E3 site of adenoviral genomic DNAs and by replacing the E1 site with an expression cassette of [CMV promoter]-attR1-[λ stuffer DNA]-attR2-[poly A attachment signal], which induced expression of the recombinant desired genomic material (see FIG. 1 and FIG.2). λ stuffer DNA was prepared to contain restriction enzyme recognition sites, such as *SwaI, ClaI* or *PacI*, by amplifying PCR primers having the restriction sites and cloning them thereto. Then, the DNA was treated with *SwaI* and/or *ClaI* to generate two DNA segments, wherein each of the segments is essential for the viral replication and packaging. The att sites had the same nucleic acid sequences as those devised by James L. et al.[see Hartley JL. et al., Genome Research, vol. 10, 1788-1795 (2000)]. pMaster vectors flanked with the mutant attL sites (attL1 and attL2) were cloned with GFP cDNA and Gus cDNA, respectively, using recombination methods (see FIG. 2, Gene-X site of pMaster vector). As the result, the plasmid vectors comprising expression cassettes of attL1-[desired genomic materials]-attL2 were generated.

DNAs of pAdHTS (1 µg) were purified using phenol/chloroform after treating them with *SwaI* and/or *ClaI* enzyme(s). The pMaster-GFP (100 ng) containing GFP cDNA, pMaster-Gus (100 ng) containing Gus cDNA, integrase (100 ng), IHF (10 ng), and Xis (10 ng) were reacted in a buffer (50mM Tris, pH 7.8, 16.5 mM NaCl, 35 mM KCl, 5 mM Spermidine, 0.25 mM EDTA, 3% Glycerol) at 25°C for 1 hr. Then, integrase, IHF and Xis were removed from E. coli according to the common method [Kotewicz M et al., Journal of Biological Chemistry, 1980, vol. 255, 2433-2439; Nash HA. and Robertson CA. Journal of Biological Chemistry, vol. 256, 9246-9253 (1981); Cho EH et al., Journal of Bacteriology, vol. 182, 5807-5812 (2000)]. The results of agarose gel electrophoretic analysis indicated that pAdHTS treated with PacI generated three DNA segments (32 kb, 4.5 kb and 1.8 kb) (FIG. 7 Lane 1). Where site-specific recombination occurred, it was observed that the segment (sized about 1.8 kb) was disappeared (FIG. 7 Lanes 2 and 3). In order to confirm this, PCR was carried out using adeno-F primer and adeno-R primer, thereby the desired sized PCR products (about 32 kb and about 4.5 kb) could be observed where the site-specific recombination was induced (see FIG. 6).

The PCR products were isolated from agarose gel, and DNA sequencing was carried out for the PCR production. As the result, it was determined that the PCR products of Lanes 2, 3 and 4 were λ DNAs resulting from insufficient cleavages of adenoviral genome. And, it was determined that the PCR product of Lane 5 was attB1-GFP-attB2, and that the PCR product of Lane 6 was attB1-Gus-attB2. That is, recombination between two adenoviral genomic segments and one pMaster clone was induced.

### EXAMPLE 2: Preparation of viruses using pAdHTS

2 micro-liter of the reaction mixture (100 ng of DNA) obtained from Example 1 was introduced to 293 packaging cells. Then, the 293 cells were transferred to 24-well (5×10⁴ cells/well) with DMEM (5% bovine fetal serum). After 24 hrs, recombination induced reaction mixture (2 micro-litter) and LipofecAmine2000 (0.5 micro-litter) (from Invitrogen Inc.) were transferred to the cells in accordance with the manufacturer's protocol. After 7 days from the transfer, CPE was observed under a microscope, which indicated the formation of viruses in the cells. At that moment, the cells were harvested and were centrifugated. The resulting precipitates were suspended in 100 micro-liter of DMEM 10% bovine fetal serum. The cells were treated at -70°C for 1 hr followed by at 37°C for 5 min. These freezing and thawing were repeated 3 times. The lysate was removed by centrifuge to produce viral mixture. Afterward, 293 cells were infected with the viral mixture. Plaque assay was carried out to detect the formation of suitable viruses.

For plaque assay, 2× 10⁵ cells were transferred to 6-well plate. After 24 hrs from the transfer, 10 micro-liter of the viral mixture mixed with 400 micro-liter of DMEM 400 was plated on the cells for 1 hr in order to induce viral adsorption. Plaques of viruses were assayed for 2 weeks, after solidifying the cells with 3 ml of DMEM to which 1% low melting-point agarose (SeaPlaque Agarose; FMC) was added. The titer of viruses was determined as the number of viral plaques per 1 ml of viral mixture. After 2 weeks, in order to detect product efficiency of the desired recombinant viruses, 500 µg of X-Gluc (5-bromo-4-chloro-3-indolil-beta-D-gluchronic acid) as substrate of Glu protein was added to the solidified agarose. After 24 hrs, the number of blue colored plaques was calculated. The titer of viruses was 10⁶∼10⁷ PFU per 1 ml. The production efficiency of recombinant viruses was determined by the recombinant viruses expressing Gus genes. All the viruses were observed as blue plaques, which suggested that 100% of the viruses obtained in the present invention were recombinant viruses. That is, only the viral genome, including desired genomic materials, replicated and formed viral particles. This had occurred because even though the efficiency of the site-specific recombination was not 100%, the separated DNA segments themselves alone and the by-products of site-specific recombination did not replicate.

As depicted in FIG. 8, recombinant viruses expressing GFP and Gus, respectively, were prepared as mentioned above. The 293 cells were infected with the recombinant viruses. Then, the expression of desired proteins (GFP or Gus) was detected under the fluorescence microscope (for GFP) or by adding X-Gluc substrate (for Gus). As shown in FIG. 8, only the group of viruses to which site-specific recombinase was added generated recombinant viruses and expressed desired proteins (see FIG. 8 Lanes 4 and 5).

### EXAMPLE 3: Preparation of recombinant viruses using pAd-Left and pAd-Right

pAd-Left (SEQ. ID No.: 4) and pAd-Right (SEQ ID No.: 5) were prepared (see FIG.3). Firstly, pAdHTS vector was treated with *PacI* and/or *SwaI*. Then, the left fragment containing LITR and packaging signals were ligated with Amp-Ori DNA from PCR amplification in order to form pAd-Left. Likewise, the right fragment (sized about 33 kb) was ligated with Amp-Ori DNA to form pAd-Right. In this study, PCR primer for Amp-Ori included ampicillin resistant gene of pBR322 DNA, a region for amplification of replication origins, and *PacI* and/or *SwaI* linker (s) for cloning restriction enzyme recognition sites. The obtained plasmid DNAs of pAd-Left and pAd-Right were generated from DH5α (Qiagen, Midiprep kit), which in turn were digested with *SwaI* and *PacI*. Using the respective plasmid DNA, site-specific recombination was induced as disclosed in Examples 1 and 2, so as to produce recombinant viruses. Briefly, linear DNAs of pAd-Left (100 ng of DNA) and linear DNA of pAd-Right (800 ng of DNA) were reacted in the presence of pMaster-GFP having GFP cDNA (100 ng), integrase (100 ng), IHF (10 ng) and Xis (10 ng) within a buffer for 1 hr. The resulting mixture was transferred to 293 packaging cells as disclosed in Example 2. After 7 days, viruses were obtained from cell debris, and plaque assay was carried out.

### EXAMPLE 4: Isolation and manipulation of AdHTS-terminal protein complex

In order to obtain AdHTS viruses, pAdHTS were digested with restriction enzyme of *PacI*, which in turn were transferred to 293 packaging cells to induce viral packaging. Mass production of AdHTS viruses was carried out by transferring about 5× 10⁵ of 293 cells to 30 cell culture dishes (150 mm of diameter) together with DMEM (5% bovine fetal serum). After 24 hrs, AdHTS virus was infected to 5 MOI. After 48 hrs from viral infection, cells were harvested from the dishes with cell scraper, and then were centrifugated. Afterward, the precipitates were suspended in a solution of 10 mM of Tris and 1mM of MgCl₂. The suspension was treated at -70°C for 1 hr followed by at 37°C for 5 min. The freezing and thawing were repeated 3 times. The cell lysate was centrifugated, and the precipitates were removed to obtain viral mixture. In order to obtain infectious viral particles, CsCl gradient ultra-centrifugation was employed. 1.4 g/ml of CsCl solution was poured into a tube (BeckMan), and then 1.2 g/ml of CsCl solution was added to the solution again. After adding 20 ml of viral mixture to the solution while maintaining the concentration gradient, the solution was centrifugated with ultra-centrifuge at 25,000 rpm for 2 hrs, thereby 1× 10¹² of infectious viral particles were isolated from defective viral particles and cell debris. Two different methods were used in order to prepare DNA-terminal protein complex from AdHTS viruses. In the first method, 6 M guanidine HCl was added to the same amount of AdHTS viral solution, and then 1 mg of ethidium bromide and saturated CSCl/4M guanidin HCl solution were added to make the final volume to 5 ml. Next, the solution was ultra-centrifugated at 650,000 g for 4 hrs. Isolated viral DNA-terminal protein complex band was detected with 365 nm of ultraviolet rays, then the DNAs corresponding to the band were extracted. The extracted DNAs were desalted for use in the following studies. In the second method, adenoviral solution, which had a mixture of 6 M guanidine HCl and the same amount of AdHTS viral solution, was transferred to superdex 16/30HR S-200 column (Amersham-Pharmacia). While developing the solution in 0.4 ml/min of flow rate, optical densities for each of the subsequent 2 ml fractions obtained continuously were detected at 260 nm and 280 nm. AdHTS DNA-terminal protein complex was detected on 1% agarose gel from the collection of peak fractions.

The AdHTS DNA-terminal protein complex obtained from above two methods was digested with *SwaI* (NEB) and stored at -70°C before being used in other studies.

### EXAMPLE 5: Plaque assays for viruses resulted from site-specific recombination with pMaster-GFP

10 ul of *in vitro* reaction mixture of pAdHTS vector from Example 2 and pMaster-GFP were transferred to 293 packaging cells as disclosed in Example 2. After 7 days, plaque assay was carried out for the viruses extracted from the cell lysate. In the same manner, plaque assay for pAd-left/pAd-right of Example 3 and for AdHTS-DNA-TPC of Example 4 were carried out, and the results are represented in Table 1 below.

**Table 1. The results of plaque assays for viruses resulted from site-specific recombination between each of adenoviral vectors and pMaster-GFP.**

| Mater Clone | pMmaster-GFP | | | |
|---|---|---|---|---|
| adenoviral vector | No recombination | PAdHTS | AdHTS-TPCC | pAd-left pAd-Right |
| total plaques (white spot) | 0 | 50 | 74 | 65 |
| GFP-positive plaques | 0 | 50 | 74 | 65 |

### EXAMPLE 6: Multi-well production of recombinant adenovirus

45 ng/well of *SwaI* treated AdHTS-DNA-terminal protein complex in Example 4 were transferred to each well of 96-well PCR plate, which in turn were subjected to site-specific recombination as in Example 1. Briefly, 100 ng of integrase, 10 ng of IHF, 10 ng of Xis, reaction buffer (50mM Tris, pH 7.8, 16.5 mM NaCl, 35 mM KCl, 5 mM spermidine, 0.25 mM EDTA, 3% glycerole), and 5 ng of pMaster-GFP including GFP cDNA were transferred to each well. As a control, 5 ng of pMaster-Gus including Gus cDNA was transferred to 4 (four) wells respectively, and reaction was continued for 1 hr. In order to confirm site-specific recombination, PCR reaction was carried out using Adeno-F primer and Adeno-R primer. As the result, PCR products corresponding to GFP were detected in 88 wells except in the controls (see FIG. 9). Similar to Example 2, 10 ul (DNA 50 ng) of the reaction mixture, which was confirmed as including site-specific recombinant, was transferred to 96-well plate, wherein each of wells contained about 5× 10³ of 292 packaging cells which were incubated for 24 hrs and wherein each of wells was coated with poly-L-lysin. The cells containing DNAs showed fluorescence. CPE was observed 4-7 days after the transfer, which indicated viral formation. At 10^{th} day from the transfer, 96-well plate was treated at -70°C for 1 hr followed by at 37°C for 5 min. The freezing and thawing were repeated 3 times to obtain cell lysate. The cell lysate was centrifugated at 2,500 rpm for 10 min. Then, the 293 cells in 96-well plate were transfected with the supernatant to confirm the formation of viruses expressing GFP (see FIG. 10). In the same manner, the expression efficiency of recombinant viruses was determined for the other adenoviral vectors, such as pAdHTS and pAd-Left/pAd-Right, in 96-well, respectively, and the average efficiency of recombinant viruses is presented on Table 2 below.

**Table 2. The expression efficieny of recombinant viruses in 96-well**

| Master clone | pMaster-GFP | | |
|---|---|---|---|
| adenoviral vector, | pAdHTS | pAd-Left/pAd-Right | AdHTS-TPC |
| expression efficiency of recombinant viruses in 96-well | 62.5 | 47.9 | 99.0 |

### INDUSTRIAL APPLICABILITIES

As disclosed above, this invention employs *in vitro* site-specific recombination in order to prepare vectors for recombinant viruses. In this invention, desired foreign genomic materials are inserted into suitable sites of viral genomic DNAs, which are digested to two or more segments, rapidly and in site-specific way. Furthermore, the reaction mixture of viral genomic DNAs and desired genomic materials can be applied to host cells *in vitro* directly without further selection of the recombinant viral genomic DNAs. Thus, according to this invention, it is possible to obtain numerous recombinant viruses at the same time. Furthermore, it can prevent self-replication of viruses almost completely. Therefore, this invention provides a high-throughput system for producing hundreds or thousands of recombinant viruses for various studies of genomes.
## Claims

1. A method for preparing linear recombinant adenoviral vectors, wherein desired DNAs are inserted into adenoviral genomic DNAs by *in vitro* site-specific recombination, **characterized in that** the method comprises:
i) providing adenoviral genomic DNA segments containing *att* site-specific recombination sites and base sequences essential to viral replication and packaging,
ii) providing carrier vectors comprising at least one desired DNAs and *att* site-specific recombination sites; and
iii) reacting the DNA segments of i) and the carrier vector of ii) *in vitro.*

2. The method for preparing linear recombinant adenoviral vectors according to claim 1, wherein the viral genomic DNA segments of i) consists of 2 (two) DNA segments which respectively are essential for viral replication and packaging, and include *att* site-specific recombination sequences.

3. The method for preparing linear recombinant adenoviral vectors according to claim 1, wherein the adenovirus is **characterized by** being deleted of E1, E2, E3, and/or E4 sites.

4. A method for preparing recombinant adenoviruses having linear genomic DNA, **characterized in that** the method comprising the steps of:
i) providing adenoviral genomic DNA segments containing *att* site-specific recombination sites and base sequences essential to viral replication and packaging;
ii) providing carrier vectors containing at least one desired DNAs and *att* site-specific recombination sequences;
iii) reacting the genomic DNA segments of i) and the carrier vectors of ii) *in vitro* using site-specific recombinase to produce mixture comprising expressive linear recombinant adenoviral vectors; and
iv) transfecting the mixture of iii) to host cells.

5. The method for preparing recombinant adenoviruses having linear genomic DNAs according to claim 4, wherein the adenoviral genomic DNAs of i) consists of 2 (two) DNA segments which respectively are essential for viral replication and packaging, and include *att* site-specific recombination sequences.

6. The method for preparing recombinant adenoviruses having linear genomic DNAs according to claim 4, wherein the adenovirus is **characterized by** being deleted of E1, E2, E3, and/or E4 site.

## Patentansprüche

1. Verfahren zum Herstellen linearer rekombinanter Adenovirusvektoren, worin die angestrebten DNAs mittels *in vitro* ortsspezifischer Rekombination in adenovirale genomische DNAs insertiert werden, **dadurch gekennzeichnet, dass** das Verfahren Folgendes umfasst:
i) Bereitstellen adenoviraler genomischer DNA-Segmente, die *att*-ortsspezifische Rekombinationsstellen und Basensequenzen enthalten, die für die Virusreplikation und -verpackung wesentlich sind,
ii) Bereitstellen von Trägervektoren, die mindestens eine der angestrebten DNAs und *att*-ortsspezifischen Rekombinationsstellen umfassen; und
iii) zur Reaktion bringen der DNA-Segmente von i) und des Trägervektors von ii) *in vitro*.

2. Verfahren zum Herstellen linearer rekombinanter Adenovirusvektoren nach Anspruch 1, worin die viralen genomischen DNA-Segmente von i) aus 2 (zwei) DNA-Segmenten bestehen, die respektive für die Virusreplikation und -verpackung wesentlich sind, und *att*-ortsspezifische Rekombinationssequenzen einschließen.

3. Verfahren zum Herstellen linearer rekombinanter Adenovirusvektoren nach Anspruch 1, worin das Adenovirus **dadurch gekennzeichnet ist, dass** die E1-, E2-, E3-und/oder E4-Stellen von ihm deletiert worden sind.

4. Verfahren zum Herstellen rekombinanter Adenoviren mit linearer genomischer DNA, **dadurch gekennzeichnet, dass** das Verfahren die folgenden Schritte umfasst:
i) Bereitstellen adenoviraler genomischer DNA-Segmente, die *att*-ortsspezifische Rekombinationsstellen und Basensequenzen enthalten, die für die Virusreplikation und -verpackung wesentlich sind;
ii) Bereitstellen von Trägervektoren, die mindestens eine der angestrebten DNAs und *att*-ortsspezifischen Rekombinationsstellen enthalten;
iii) zur Reaktion bringen der genomischen DNA-Segmente von i) und der Trägervektoren von ii) *in vitro* unter Verwendung von ortsspezifischer Rekombinase zum Herstellen eines Gemischs, das expressive, lineare rekombinante Adenovektoren umfasst; und
iv) Transfizieren des Gemischs von iii) in Wirtszellen.

5. Verfahren zum Herstellen rekombinanter Adenoviren mit linearen genomischen DNAs nach Anspruch 4, worin die adenoviralen genomischen DNAs von i) aus 2 (zwei) DNA-Segmenten bestehen, die respektive für die Virusreplikation und -verpackung wesentlich sind, und *att*-ortsspezifische Rekombinationssequenzen einschließen.

6. Verfahren zum Herstellen rekombinanter Adenoviren mit linearen genomischen DNAs nach Anspruch 4, worin das Adenovirus **dadurch gekennzeichnet ist, dass** die E1-, E2-, E3- und/oder E4-Stelle von ihm deletiert worden ist.

## Revendications

1. Méthode de préparation de vecteurs adénoviraux recombinés linéaires, dans laquelle des ADN désirés sont insérés dans des ADN génomiques adénoviraux par recombinaison site-spécifique *in vitro*, **caractérisée en ce que** la méthode comprend les étapes consistant à :
i) fournir des segments d'ADN génomique adénoviral contenant des sites de recombinaison spécifiques de site *att* et des séquences de bases essentielles à la réplication et l'encapsidation virales,
ii) fournir des vecteurs supports comprenant au moins un ADN désiré et des sites de recombinaison spécifiques de site *att*; et
iii) faire réagir les segments d'ADN de i) et le vecteur support de ii) *in vitro*.

2. Méthode de préparation de vecteurs adénoviraux recombinés linéaires selon la revendication 1, dans laquelle les segments d'ADN génomique viral de i) sont constitués de 2 (deux) segments d'ADN qui respectivement sont essentiels à la réplication et l'encapsidation virales, et comportent des séquences de recombinaison spécifiques de site *att*.

3. Méthode de préparation de vecteurs adénoviraux recombinés linéaires selon la revendication 1, dans laquelle l'adénovirus est **caractérisé par** la délétion de ses sites E1, E2, E3 et/ou E4.

4. Méthode de préparation d'adénovirus recombinés ayant un ADN génomique linéaire, **caractérisée en ce que** la méthode comprend les étapes consistant à :
i) fournir des segments d'ADN génomique adénoviral contenant des sites de recombinaison spécifiques de site *att* et des séquences de bases essentielles à la réplication et l'encapsidation virales,
ii) fournir des vecteurs supports contenant au moins un ADN désiré et des séquences de recombinaison spécifiques de site *att* ;
iii) faire réagir les segments d'ADN génomique de i) et les vecteurs supports de ii) *in vitro* à l'aide de recombinase site-spécifique afin de produire un mélange comprenant des vecteurs adénoviraux recombinés linéaires expressifs ; et
iv) transfecter le mélange de iii) dans des cellules hôtes.

5. Méthode de préparation d'adénovirus recombinés ayant des ADN génomiques linéaires selon la revendication 4, dans laquelle les ADN génomiques adénoviraux de i) sont constitués de 2 (deux) segments d'ADN qui respectivement sont essentiels à la réplication et l'encapsidation virales, et comportent des séquences de recombinaison spécifiques de site *att*.

6. Méthode de préparation d'adénovirus recombinés ayant des ADN génomiques linéaires selon la revendication 4, dans laquelle l'adénovirus est **caractérisé par** la délétion de ses sites E1, E2, E3 et/ou E4.


<110> Neurogenex Co., Ltd.
<120> A METHOD FOR PRODUCING RECOMBINANT VIRUSES USING SITE-SPECIFIC RECOMBINATION
<130> PN0022781.00
<150> KR10-2002-0012633
   <151> 2002-03-09
<160> 5
<170> KopatentIn 1.71
<210> 1
   <211> 34573
   <212> DNA
   <213> Artificial Sequence
<220>
   <223> pAdHTS vector
<220>
   <221> misc_feature
   <222> (3181)..(30681)
   <223> Ad5
<220>
   <221> promoter
   <222> (400)..(930)
   <223> CMV promoter
<220>
   <221> misc_feature
   <222> (33318)..(34112)
   <223> kanamycin
<220>
   <221> rep_origin
   <222> (31716)..(32493)
   <223> pBR322 ori
<220>
   <221> misc_feature
   <222> (31536)..(31638)
   <223> RITR
<220>
   <221> polyA_site
   <222> (1747)..(1868)
   <223> SV40 polyA
<220>
   <221> misc_feature
   <222> (197)..(345)
   <223> Ad5 psi
<220>
   <221> misc_feature
   <222> (1464)..(1588)
   <223> attR2 site
<220>
   <221> misc_feature
   <222> (949)..(1073)
   <223> attR1 site
<400> 1
<210> 2
   <211> 20
   <212> DNA
   <213> Artificial Sequence
<220>
   <223> Adeno-F primer
<400> 2
   ggtctatata agcagagctg 20
<210> 3
   <211> 20
   <212> DNA
   <213> Artificial Sequence
<220>
   <223> Adeno-R primer
<400> 3
   gtatggctga ttatgatcag 20
<210> 4
   <211> 4752
   <212> DNA
   <213> Artificial Sequence
<220>
   <223> pAd-Left
<220>
   <221> misc_feature
   <222> (1227)..(2087)
   <223> R Amp
<220>
   <221> promoter
   <222> (400)..(930)
   <223> F CMV promoter
<220>
   <221> rep_origin
   <222> (2235)..(2902)
   <223> R pBR322 Ori
<220>
   <221> misc_feature
   <222> (197)..(345)
   <223> F Ad5 psi
<220>
   <221> misc_feature
   <222> (4)..(106)
   <223> F LITR
<220>
   <221> misc_feature
   <222> (949)..(1073)
   <223> F attR1
<400> 4
   taacatcatc aataatatac cttattttgg attgaagcca atatgataat gagggggtgg 60
   agtttgtgac gtggcgcggg gcgtgggaac ggggcgggtg acgtagtagt gtggcggaag 120
   tgtgatgttg caagtgtggc ggaacacatg taagcgacgg atgtggcaaa agtgacgttt 180
   ttggtgtgcg ccggtgtaca caggaagtga caattttcgc gcggttttag gcggatgttg 240
   tagtaaattt gggcgtaacc gagtaagatt tggccatttt cgcgggaaaa ctgaataaga 300
   ggaagtgaaa tctgaataat tttgtgttac tcatagcgcg taatactgta atagtaatca 360
   attacggggt cattagttca tagcccatat atggagttcc gcgttacata acttacggta 420
   aatggcccgc ctggctgacc gcccaacgac ccccgcccat tgacgtcaat aatgacgtat 480
   gttcccatag taacgccaat agggactttc cattgacgtc aatgggtgga gtatttacgg 540
   taaactgccc acttggcagt acatcaagtg tatcatatgc caagtacgcc ccctattgac 600
   gtcaatgacg gtaaatggcc cgcctggcat tatgcccagt acatgacctt atgggacttt 660
   cctacttggc agtacatcta cgtattagtc atcgctatta ccatggtgat gcggttttgg 720
   cagtacatca atgggcgtgg atagcggttt gactcacggg gatttccaag tctccacccc 780
   attgacgtca atgggagttt gttttggcac caaaatcaac gggactttcc aaaatgtcgt 840
   aacaactccg ccccattgac gcaaatgggc ggtaggcgtg tacggtggga ggtctatata 900
   agcagagctg gtttagtgaa ccgtcagatc cgctagagat ctggtaccac aagtttgtac 960
   aaaaaagctg aacgagaaac gtaaaatgat ataaatatca atatattaaa ttagattttg 1020
   cataaaaaac agactacata atactgtaaa acacaacata tccagtcact atggcggccg 1080
   catttaaata tcgatcaggt ggcacttttc ggggaaatgt gcgcggaacc cctatttgtt 1140
   tatttttcta aatacattca aatatgtatc cgctcatgag acaataaccc tgataaatgc 1200
   ttcaataata ttgaaaaagg aagagtatga gtattcaaca tttccgtgtc gcccttattc 1260
   ccttttttgc ggcattttgc cttcctgttt ttgctcaccc agaaacgctg gtgaaagtaa 1320
   aagatgctga agatcagttg ggtgcacgag tgggttacat cgaactggat ctcaacagcg 1380
   gtaagatcct tgagagtttt cgccccgaag aacgttttcc aatgatgagc acttttaaag 1440
   ttctgctatg tggcgcggta ttatcccgtg ttgacgccgg gcaagagcaa ctcggtcgcc 1500
   gcatacacta ttctcagaat gacttggttg agtactcacc agtcacagaa aagcatctta 1560
   cggatggcat gacagtaaga gaattatgca gtgctgccat aaccatgagt gataacactg 1620
   cggccaactt acttctgaca acgatcggag gaccgaagga gctaaccgct tttttgcaca 1680
   acatggggga tcatgtaact cgccttgatc gttgggaacc ggagctgaat gaagccatac 1740
   caaacgacga gcgtgacacc acgatgcctg cagcaatggc aacaacgttg cgcaaactat 1800
   taactggcga actacttact ctagcttccc ggcaacaatt aatagactgg atggaggcgg 1860
   ataaagttgc aggaccactt ctgcgctcgg cccttccggc tggctggttt attgctgata 1920
   aatctggagc cggtgagcgt gggtctcgcg gtatcattgc agcactgggg ccagatggta 1980
   agccctcccg tatcgtagtt atctacacga cggggagtca ggcaactatg gatgaacgaa 2040
   atagacagat cgctgagata ggtgcctcac tgattaagca ttggtaactg tcagaccaag 2100
   tttactcata tatactttag attgatttaa aacttcattt ttaatttaaa aggatctagg 2160
   tgaagatcct ttttgataat ctcatgacca aaatccctta acgtgagttt tcgttccact 2220
   gagcgtcaga ccccgtagaa aagatcaaag gatcttcttg agatcctttt tttctgcgcg 2280
   taatctgctg cttgcaaaca aaaaaaccac cgctaccagc ggtggtttgt ttgccggatc 2340
   aagagctacc aactcttttt ccgaaggtaa ctggcttcag cagagcgcag ataccaaata 2400
   ctgtccttct agtgtagccg tagttaggcc accacttcaa gaactctgta gcaccgccta 2460
   catacctcgc tctgctaatc ctgttaccag tggctgctgc cagtggcgat aagtcgtgtc 2520
   ttaccgggtt ggactcaaga cgatagttac cggataaggc gcagcggtcg ggctgaacgg 2580
   ggggttcgtg cacacagccc agcttggagc gaacgaccta caccgaactg agatacctac 2640
   agcgtgagca ttgagaaagc gccacgcttc ccgaagggag aaaggcggac aggtatccgg 2700
   taagcggcag ggtcggaaca ggagagcgca cgagggagct tccaggggga aacgcctggt 2760
   atctttatag tcctgtcggg tttcgccacc tctgacttga gcgtcgattt ttgtgatgct 2820
   cgtcaggggg gcggagccta tggaaaaacg ccagcaacgc ggccttttta cggttcctgg 2880
   ccttttgctg gccttttgct cacatgttct ttcctgcgtt atcccctgat tctgtggata 2940
   accgtattac cgcctttgag tgagctgata ccgctcgccg cagccgaacg accgagcgca 3000
   gcgagtcagt gagcgaggaa gcggaagagc gcctgatgcg gtattttctc cttacgcatc 3060
   tgtgcggtat ttcacaccgc atatggtgca ctctcagtac aatctgctct gatgccgcat 3120
   agttaagcca gtatacactc cgctatcgct acgtgactgg gtcatggctg cgccccgaca 3180
   cccgccaaca cccgctgacg cgccctgacg ggcttgtctg ctcccggcat ccgcttacag 3240
   acaagctgtg accgtctccg ggagctgcat gtgtcagagg ttttcaccgt catcaccgaa 3300
   acgcgcgagg cagctgcggt aaagctcatc agcgtggtcg tgaagcgatt cacagatgtc 3360
   tgcctgttca tccgcgtcca gctcgttgag tttctccaga agcgttaatg tctggcttct 3420
   gataaagcgg gccatgttaa gggcggtttt ttcctgtttg gtcacttgat gcctccgtgt 3480
   aagggggaat ttctgttcat gggggtaatg ataccgatga aacgagagag gatgctcacg 3540
   atacgggtta ctgatgatga acatgcccgg ttactggaac gttgtgaggg taaacaactg 3600
   gcggtatgga tgcggcggga ccagagaaaa atcactcagg gtcaatgcca gcgcttcgtt 3660
   aatacagatg taggtgttcc acagggtagc cagcagcatc ctgcgatgca gatccggaac 3720
   ataatggtgc agggcgctga cttccgcgtt tccagacttt acgaaacacg gaaaccgaag 3780
   accattcatg ttgttgctca ggtcgcagac gttttgcagc agcagtcgct tcacgttcgc 3840
   tcgcgtatcg gtgattcatt ctgctaacca gtaaggcaac cccgccagcc tagccgggtc 3900
   ctcaacgaca ggagcacgat catgcgcacc cgtggccagg acccaacgct gcccgagatg 3960
   cgccgcgtgc ggctgctgga gatggcggac gcgatggata tgttctgcca agggttggtt 4020
   tgcgcattca cagttctccg caagaattga ttggctccaa ttcttggagt ggtgaatccg 4080
   ttagcgaggt gccgccggct tccattcagg tcgaggtggc ccggctccat gcaccgcgac 4140
   gcaacgcggg gaggcagaca aggtataggg cggcgcctac aatccatgcc aacccgttcc 4200
   atgtgctcgc cgaggcggca taaatcgccg tgacgatcag cggtccagtg atcgaagtta 4260
   ggctggtaag agccgcgagc gatccttgaa gctgtccctg atggtcgtca tctacctgcc 4320
   tggacagcat ggcctgcaac gcgggcatcc cgatgccgcc ggaagcgaga agaatcataa 4380
   tggggaaggc catccagcct cgcgtcgcga acgccagcaa gacgtagccc agcgcgtcgg 4440
   ccgccatgcc ggcgataatg gcctgcttct cgccgaaacg tttggtggcg ggaccagtga 4500
   cgaaggcttg agcgagggcg tgcaagattc cgaataccgc aagcgacagg ccgatcatcg 4560
   tcgcgctcca gcgaaagcgg tcctcgccga aaatgaccca gagcgctgcc ggcacctgtc 4620
   ctacgagttg catgataaag aagacagtca taagtgcggc gacgatagtc atgccccgcg 4680
   cccaccggaa ggagctgact gggttgaagg ctctcaaggg catcgtcgag acgaggatcc 4740
   atgcatgtta at 4752
<210> 5
   <211> 33855
   <212> DNA
   <213> Artificial Sequence
<220>
   <223> pAd-Right
<220>
   <221> source
   <222> (1)..(3657)
   <223> F pAd-Easyl
<220>
   <221> misc_feature
   <222> (2666)..(3526)
   <223> F Amp
<220>
   <221> rep_origin
   <222> (1851)..(2518)
   <223> F pBR322 Ori
<220>
   <221> misc_feature
   <222> (33750)..(33852)
   <223> F RITR
<220>
   <221> polyA_site
   <222> (3961)..(4082)
   <223> F SV40 polyA
<220>
   <221> misc_feature
   <222> (3678)..(3802)
   <223> R attR2
<400> 5
   attaacatgc atggatcctc gtctcgacga tgcccttgag agccttcaac ccagtcagct 60
   ccttccggtg ggcgcggggc atgactatcg tcgccgcact tatgactgtc ttctttatca 120
   tgcaactcgt aggacaggtg ccggcagcgc tctgggtcat tttcggcgag gaccgctttc 180
   gctggagcgc gacgatgatc ggcctgtcgc ttgcggtatt cggaatcttg cacgccctcg 240
   ctcaagcctt cgtcactggt cccgccacca aacgtttcgg cgagaagcag gccattatcg 300
   ccggcatggc ggccgacgcg ctgggctacg tcttgctggc gttcgcgacg cgaggctgga 360
   tggccttccc cattatgatt cttctcgctt ccggcggcat cgggatgccc gcgttgcagg 420
   ccatgctgtc caggcaggta gatgacgacc atcagggaca gcttcaagga tcgctcgcgg 480
   ctcttaccag cctaacttcg atcactggac cgctgatcgt cacggcgatt tatgccgcct 540
   cggcgagcac atggaacggg ttggcatgga ttgtaggcgc cgccctatac cttgtctgcc 600
   tccccgcgtt gcgtcgcggt gcatggagcc gggccacctc gacctgaatg gaagccggcg 660
   gcacctcgct aacggattca ccactccaag aattggagcc aatcaattct tgcggagaac 720
   tgtgaatgcg caaaccaacc cttggcagaa catatccatc gcgtccgcca tctccagcag 780
   ccgcacgcgg cgcatctcgg gcagcgttgg gtcctggcca cgggtgcgca tgatcgtgct 840
   cctgtcgttg aggacccggc taggctggcg gggttgcctt actggttagc agaatgaatc 900
   accgatacgc gagcgaacgt gaagcgactg ctgctgcaaa acgtctgcga cctgagcaac 960
   aacatgaatg gtcttcggtt tccgtgtttc gtaaagtctg gaaacgcgga agtcagcgcc 1020
   ctgcaccatt atgttccgga tctgcatcgc aggatgctgc tggctaccct gtggaacacc 1080
   tacatctgta ttaacgaagc gctggcattg accctgagtg atttttctct ggtcccgccg 1140
   catccatacc gccagttgtt taccctcaca acgttccagt aaccgggcat gttcatcatc 1200
   agtaacccgt atcgtgagca tcctctctcg tttcatcggt atcattaccc ccatgaacag 1260
   aaattccccc ttacacggag gcatcaagtg accaaacagg aaaaaaccgc ccttaacatg 1320
   gcccgcttta tcagaagcca gacattaacg cttctggaga aactcaacga gctggacgcg 1380
   gatgaacagg cagacatctg tgaatcgctt cacgaccacg ctgatgagct ttaccgcagc 1440
   tgcctcgcgc gtttcggtga tgacggtgaa aacctctgac acatgcagct cccggagacg 1500
   gtcacagctt gtctgtaagc ggatgccggg agcagacaag cccgtcaggg cgcgtcagcg 1560
   ggtgttggcg ggtgtcgggg cgcagccatg acccagtcac gtagcgatag cggagtgtat 1620
   actggcttaa ctatgcggca tcagagcaga ttgtactgag agtgcaccat atgcggtgtg 1680
   aaataccgca cagatgcgta aggagaaaat accgcatcag gcgctcttcc gcttcctcgc 1740
   tcactgactc gctgcgctcg gtcgttcggc tgcggcgagc ggtatcagct cactcaaagg 1800
   cggtaatacg gttatccaca gaatcagggg ataacgcagg aaagaacatg tgagcaaaag 1860
   gccagcaaaa ggccaggaac cgtaaaaagg ccgcgttgct ggcgtttttc cataggctcc 1920
   gcccccctga cgagcatcac aaaaatcgac gctcaagtca gaggtggcga aacccgacag 1980
   gactataaag ataccaggcg tttccccctg gaagctccct cgtgcgctct cctgttccga 2040
   ccctgccgct taccggatac ctgtccgcct ttctcccttc gggaagcgtg gcgctttctc 2100
   aatgctcacg ctgtaggtat ctcagttcgg tgtaggtcgt tcgctccaag ctgggctgtg 2160
   tgcacgaacc ccccgttcag cccgaccgct gcgccttatc cggtaactat cgtcttgagt 2220
   ccaacccggt aagacacgac ttatcgccac tggcagcagc cactggtaac aggattagca 2280
   gagcgaggta tgtaggcggt gctacagagt tcttgaagtg gtggcctaac tacggctaca 2340
   ctagaaggac agtatttggt atctgcgctc tgctgaagcc agttaccttc ggaaaaagag 2400
   ttggtagctc ttgatccggc aaacaaacca ccgctggtag cggtggtttt tttgtttgca 2460
   agcagcagat tacgcgcaga aaaaaaggat ctcaagaaga tcctttgatc ttttctacgg 2520
   ggtctgacgc tcagtggaac gaaaactcac gttaagggat tttggtcatg agattatcaa 2580
   aaaggatctt cacctagatc cttttaaatt aaaaatgaag ttttaaatca atctaaagta 2640
   tatatgagta aacttggtct gacagttacc aatgcttaat cagtgaggca cctatctcag 2700
   cgatctgtct atttcgttca tccatagttg cctgactccc cgtcgtgtag ataactacga 2760
   tacgggaggg cttaccatct ggccccagtg ctgcaatgat accgcgagac ccacgctcac 2820
   cggctccaga tttatcagca ataaaccagc cagccggaag ggccgagcgc agaagtggtc 2880
   ctgcaacttt atccgcctcc atccagtcta ttaattgttg ccgggaagct agagtaagta 2940
   gttcgccagt taatagtttg cgcaacgttg ttgccattgc tgcaggcatc gtggtgtcac 3000
   gctcgtcgtt tggtatggct tcattcagct ccggttccca acgatcaagg cgagttacat 3060
   gatcccccat gttgtgcaaa aaagcggtta gctccttcgg tcctccgatc gttgtcagaa 3120
   gtaagttggc cgcagtgtta tcactcatgg ttatggcagc actgcataat tctcttactg 3180
   tcatgccatc cgtaagatgc ttttctgtga ctggtgagta ctcaaccaag tcattctgag 3240
   aatagtgtat gcggcgaccg agttgctctt gcccggcgtc aacacgggat aataccgcgc 3300
   cacatagcag aactttaaaa gtgctcatca ttggaaaacg ttcttcgggg cgaaaactct 3360
   caaggatctt accgctgttg agatccagtt cgatgtaacc cactcgtgca cccaactgat 3420
   cttcagcatc ttttactttc accagcgttt ctgggtgagc aaaaacagga aggcaaaatg 3480
   ccgcaaaaaa gggaataagg gcgacacgga aatgttgaat actcatactc ttcctttttc 3540
   aatattattg aagcatttat cagggttatt gtctcatgag cggatacata tttgaatgta 3600
   tttagaaaaa taaacaaata ggggttccgc gcacatttcc ccgaaaagtg ccacctgatc 3660
   gatatttaaa tgtcgaccat agtgactgga tatgttgtgt tttacagtat tatgtagtct 3720
   gttttttatg caaaatctaa tttaatatat tgatatttat atcattttac gtttctcgtt 3780
   cagctttctt gtacaaagtg gtctcgagcc taagcttcta gataagatat ccgatccacc 3840
   ggatctagat aactgatcat aatcagccat accacatttg tagaggtttt acttgcttta 3900
   aaaaacctcc cacacctccc cctgaacctg aaacataaaa tgaatgcaat tgttgttgtt 3960
   aacttgttta ttgcagctta taatggttac aaataaagca atagcatcac aaatttcaca 4020
   aataaagcat ttttttcact gcattctagt tgtggtttgt ccaaactcat caatgtatct 4080
   taacgcggat ctgggcgtgg ttaagggtgg gaaagaatat ataaggtggg ggtcttatgt 4140
   agttttgtat ctgttttgca gcagccgccg ccgccatgag caccaactcg tttgatggaa 4200
   gcattgtgag ctcatatttg acaacgcgca tgcccccatg ggccggggtg cgtcagaatg 4260
   tgatgggctc cagcattgat ggtcgccccg tcctgcccgc aaactctact accttgacct 4320
   acgagaccgt gtctggaacg ccgttggaga ctgcagcctc cgccgccgct tcagccgctg 4380
   cagccaccgc ccgcgggatt gtgactgact ttgctttcct gagcccgctt gcaagcagtg 4440
   cagcttcccg ttcatccgcc cgcgatgaca agttgacggc tcttttggca caattggatt 4500
   ctttgacccg ggaacttaat gtcgtttctc agcagctgtt ggatctgcgc cagcaggttt 4560
   ctgccctgaa ggcttcctcc cctcccaatg cggtttaaaa cataaataaa aaaccagact 4620
   ctgtttggat ttggatcaag caagtgtctt gctgtcttta tttaggggtt ttgcgcgcgc 4680
   ggtaggcccg ggaccagcgg tctcggtcgt tgagggtcct gtgtattttt tccaggacgt 4740
   ggtaaaggtg actctggatg ttcagataca tgggcataag cccgtctctg gggtggaggt 4800
   agcaccactg cagagcttca tgctgcgggg tggtgttgta gatgatccag tcgtagcagg 4860
   agcgctgggc gtggtgccta aaaatgtctt tcagtagcaa gctgattgcc aggggcaggc 4920
   ccttggtgta agtgtttaca aagcggttaa gctgggatgg gtgcatacgt ggggatatga 4980
   gatgcatctt ggactgtatt tttaggttgg ctatgttccc agccatatcc ctccggggat 5040
   tcatgttgtg cagaaccacc agcacagtgt atccggtgca cttgggaaat ttgtcatgta 5100
   gcttagaagg aaatgcgtgg aagaacttgg agacgccctt gtgacctcca agattttcca 5160
   tgcattcgtc cataatgatg gcaatgggcc cacgggcggc ggcctgggcg aagatatttc 5220
   tgggatcact aacgtcatag ttgtgttcca ggatgagatc gtcataggcc atttttacaa 5280
   agcgcgggcg gagggtgcca gactgcggta taatggttcc atccggccca ggggcgtagt 5340
   taccctcaca gatttgcatt tcccacgctt tgagttcaga tggggggatc atgtctacct 5400
   gcggggcgat gaagaaaacg gtttccgggg taggggagat cagctgggaa gaaagcaggt 5460
   tcctgagcag ctgcgactta ccgcagccgg tgggcccgta aatcacacct attaccgggt 5520
   gcaactggta gttaagagag ctgcagctgc cgtcatccct gagcaggggg gccacttcgt 5580
   taagcatgtc cctgactcgc atgttttccc tgaccaaatc cgccagaagg cgctcgccgc 5640
   ccagcgatag cagttcttgc aaggaagcaa agtttttcaa cggtttgaga ccgtccgccg 5700
   taggcatgct tttgagcgtt tgaccaagca gttccaggcg gtcccacagc tcggtcacct 5760
   gctctacggc atctcgatcc agcatatctc ctcgtttcgc gggttggggc ggctttcgct 5820
   gtacggcagt agtcggtgct cgtccagacg ggccagggtc atgtctttcc acgggcgcag 5880
   ggtcctcgtc agcgtagtct gggtcacggt gaaggggtgc gctccgggct gcgcgctggc 5940
   cagggtgcgc ttgaggctgg tcctgctggt gctgaagcgc tgccggtctt cgccctgcgc 6000
   gtcggccagg tagcatttga ccatggtgtc atagtccagc ccctccgcgg cgtggccctt 6060
   ggcgcgcagc ttgcccttgg aggaggcgcc gcacgagggg cagtgcagac ttttgagggc 6120
   gtagagcttg ggcgcgagaa ataccgattc cggggagtag gcatccgcgc cgcaggcccc 6180
   gcagacggtc tcgcattcca cgagccaggt gagctctggc cgttcggggt caaaaaccag 6240
   gtttccccca tgctttttga tgcgtttctt acctctggtt tccatgagcc ggtgtccacg 6300
   ctcggtgacg aaaaggctgt ccgtgtcccc gtatacagac ttgagaggcc tgtcctcgag 6360
   cggtgttccg cggtcctcct cgtatagaaa ctcggaccac tctgagacaa aggctcgcgt 6420
   ccaggccagc acgaaggagg ctaagtggga ggggtagcgg tcgttgtcca ctagggggtc 6480
   cactcgctcc agggtgtgaa gacacatgtc gccctcttcg gcatcaagga aggtgattgg 6540
   tttgtaggtg taggccacgt gaccgggtgt tcctgaaggg gggctataaa agggggtggg 6600
   ggcgcgttcg tcctcactct cttccgcatc gctgtctgcg agggccagct gttggggtga 6660
   gtactccctc tgaaaagcgg gcatgacttc tgcgctaaga ttgtcagttt ccaaaaacga 6720
   ggaggatttg atattcacct ggcccgcggt gatgcctttg agggtggccg catccatctg 6780
   gtcagaaaag acaatctttt tgttgtcaag cttggtggca aacgacccgt agagggcgtt 6840
   ggacagcaac ttggcgatgg agcgcagggt ttggtttttg tcgcgatcgg cgcgctcctt 6900
   ggccgcgatg tttagctgca cgtattcgcg cgcaacgcac cgccattcgg gaaagacggt 6960
   ggtgcgctcg tcgggcacca ggtgcacgcg ccaaccgcgg ttgtgcaggg tgacaaggtc 7020
   aacgctggtg gctacctctc cgcgtaggcg ctcgttggtc cagcagaggc ggccgccctt 7080
   gcgcgagcag aatggcggta gggggtctag ctgcgtctcg tccggggggt ctgcgtccac 7140
   ggtaaagacc ccgggcagca ggcgcgcgtc gaagtagtct atcttgcatc cttgcaagtc 7200
   tagcgcctgc tgccatgcgc gggcggcaag cgcgcgctcg tatgggttga gtgggggacc 7260
   ccatggcatg gggtgggtga gcgcggaggc gtacatgccg caaatgtcgt aaacgtagag 7320
   gggctctctg agtattccaa gatatgtagg gtagcatctt ccaccgcgga tgctggcgcg 7380
   cacgtaatcg tatagttcgt gcgagggagc gaggaggtcg ggaccgaggt tgctacgggc 7440
   gggctgctct gctcggaaga ctatctgcct gaagatggca tgtgagttgg atgatatggt 7500
   tggacgctgg aagacgttga agctggcgtc tgtgagacct accgcgtcac gcacgaagga 7560
   ggcgtaggag tcgcgcagct tgttgaccag ctcggcggtg acctgcacgt ctagggcgca 7620
   gtagtccagg gtttccttga tgatgtcata cttatcctgt cccttttttt tccacagctc 7680
   gcggttgagg acaaactctt cgcggtcttt ccagtactct tggatcggaa acccgtcggc 7740
   ctccgaacgg taagagccta gcatgtagaa ctggttgacg gcctggtagg cgcagcatcc 7800
   cttttctacg ggtagcgcgt atgcctgcgc ggccttccgg agcgaggtgt gggtgagcgc 7860
   aaaggtgtcc ctgaccatga ctttgaggta ctggtatttg aagtcagtgt cgtcgcatcc 7920
   gccctgctcc cagagcaaaa agtccgtgcg ctttttggaa cgcggatttg gcagggcgaa 7980
   ggtgacatcg ttgaagagta tctttcccgc gcgaggcata aagttgcgtg tgatgcggaa 8040
   gggtcccggc acctcggaac ggttgttaat tacctgggcg gcgagcacga tctcgtcaaa 8100
   gccgttgatg ttgtggccca caatgtaaag ttccaagaag cgcgggatgc ccttgatgga 8160
   aggcaatttt ttaagttcct cgtaggtgag ctcttcaggg gagctgagcc cgtgctctga 8220
   aagggcccag tctgcaagat gagggttgga agcgacgaat gagctccaca ggtcacgggc 8280
   cattagcatt tgcaggtggt cgcgaaaggt cctaaactgg cgacctatgg ccattttttc 8340
   tggggtgatg cagtagaagg taagcgggtc ttgttcccag cggtcccatc caaggttcgc 8400
   ggctaggtct cgcgcggcag tcactagagg ctcatctccg ccgaacttca tgaccagcat 8460
   gaagggcacg agctgcttcc caaaggcccc catccaagta taggtctcta catcgtaggt 8520
   gacaaagaga cgctcggtgc gaggatgcga gccgatcggg aagaactgga tctcccgcca 8580
   ccaattggag gagtggctat tgatgtggtg aaagtagaag tccctgcgac gggccgaaca 8640
   ctcgtgctgg cttttgtaaa aacgtgcgca gtactggcag cggtgcacgg gctgtacatc 8700
   ctgcacgagg ttgacctgac gaccgcgcac aaggaagcag agtgggaatt tgagcccctc 8760
   gcctggcggg tttggctggt ggtcttctac ttcggctgct tgtccttgac cgtctggctg 8820
   ctcgagggga gttacggtgg atcggaccac cacgccgcgc gagcccaaag tccagatgtc 8880
   cgcgcgcggc ggtcggagct tgatgacaac atcgcgcaga tgggagctgt ccatggtctg 8940
   gagctcccgc ggcgtcaggt caggcgggag ctcctgcagg tttacctcgc atagacgggt 9000
   cagggcgcgg gctagatcca ggtgatacct aatttccagg ggctggttgg tggcggcgtc 9060
   gatggcttgc aagaggccgc atccccgcgg cgcgactacg gtaccgcgcg gcgggcggtg 9120
   ggccgcgggg gtgtccttgg atgatgcatc taaaagcggt gacgcgggcg agcccccgga 9180
   ggtagggggg gctccggacc cgccgggaga gggggcaggg gcacgtcggc gccgcgcgcg 9240
   ggcaggagct ggtgctgcgc gcgtaggttg ctggcgaacg cgacgacgcg gcggttgatc 9300
   tcctgaatct ggcgcctctg cgtgaagacg acgggcccgg tgagcttgag cctgaaagag 9360
   agttcgacag aatcaatttc ggtgtcgttg acggcggcct ggcgcaaaat ctcctgcacg 9420
   tctcctgagt tgtcttgata ggcgatctcg gccatgaact gctcgatctc ttcctcctgg 9480
   agatctccgc gtccggctcg ctccacggtg gcggcgaggt cgttggaaat gcgggccatg    9540
   agctgcgaga aggcgttgag gcctccctcg ttccagacgc ggctgtagac cacgccccct    9600
   tcggcatcgc gggcgcgcat gaccacctgc gcgagattga gctccacgtg ccgggcgaag 9660
   acggcgtagt ttcgcaggcg ctgaaagagg tagttgaggg tggtggcggt gtgttctgcc 9720
   acgaagaagt acataaccca gcgtcgcaac gtggattcgt tgatatcccc caaggcctca 9780
   aggcgctcca tggcctcgta gaagtccacg gcgaagttga aaaactggga gttgcgcgcc 9840
   gacacggtta actcctcctc cagaagacgg atgagctcgg cgacagtgtc gcgcacctcg 9900
   cgctcaaagg ctacaggggc ctcttcttct tcttcaatct cctcttccat aagggcctcc 9960
   ccttcttctt cttctggcgg cggtggggga ggggggacac ggcggcgacg acggcgcacc 10020
   gggaggcggt cgacaaagcg ctcgatcatc tccccgcggc gacggcgcat ggtctcggtg 10080
   acggcgcggc cgttctcgcg ggggcgcagt tggaagacgc cgcccgtcat gtcccggtta 10140
   tgggttggcg gggggctgcc atgcggcagg gatacggcgc taacgatgca tctcaacaat 10200
   tgttgtgtag gtactccgcc gccgagggac ctgagcgagt ccgcatcgac cggatcggaa 10260
   aacctctcga gaaaggcgtc taaccagtca cagtcgcaag gtaggctgag caccgtggcg 10320
   ggcggcagcg ggcggcggtc ggggttgttt ctggcggagg tgctgctgat gatgtaatta 10380
   aagtaggcgg tcttgagacg gcggatggtc gacagaagca ccatgtcctt gggtccggcc 10440
   tgctgaatgc gcaggcggtc ggccatgccc caggcttcgt tttgacatcg gcgcaggtct 10500
   ttgtagtagt cttgcatgag cctttctacc ggcacttctt cttctccttc ctcttgtcct 10560
   gcatctcttg catctatcgc tgcggcggcg gcggagtttg gccgtaggtg gcgccctctt 10620
   cctcccatgc gtgtgacccc gaagcccctc atcggctgaa gcagggctag gtcggcgaca 10680
   acgcgctcgg ctaatatggc ctgctgcacc tgcgtgaggg tagactggaa gtcatccatg 10740
   tccacaaagc ggtggtatgc gcccgtgttg atggtgtaag tgcagttggc cataacggac 10800
   cagttaacgg tctggtgacc cggctgcgag agctcggtgt acctgagacg cgagtaagcc 10860
   ctcgagtcaa atacgtagtc gttgcaagtc cgcaccaggt actggtatcc caccaaaaag 10920
   tgcggcggcg gctggcggta gaggggccag cgtagggtgg ccggggctcc gggggcgaga 10980
   tcttccaaca taaggcgatg atatccgtag atgtacctgg acatccaggt gatgccggcg 11040
   gcggtggtgg aggcgcgcgg aaagtcgcgg acgcggttcc agatgttgcg cagcggcaaa 11100
   aagtgctcca tggtcgggac gctctggccg gtcaggcgcg cgcaatcgtt gacgctctac 11160
   cgtgcaaaag gagagcctgt aagcgggcac tcttccgtgg tctggtggat aaattcgcaa 11220
   gggtatcatg gcggacgacc ggggttcgag ccccgtatcc ggccgtccgc cgtgatccat 11280
   gcggttaccg cccgcgtgtc gaacccaggt gtgcgacgtc agacaacggg ggagtgctcc 11340
   ttttggcttc cttccaggcg cggcggctgc tgcgctagct tttttggcca ctggccgcgc 11400
   gcagcgtaag cggttaggct ggaaagcgaa agcattaagt ggctcgctcc ctgtagccgg 11460
   agggttattt tccaagggtt gagtcgcggg acccccggtt cgagtctcgg accggccgga 11520
   ctgcggcgaa cgggggtttg cctccccgtc atgcaagacc ccgcttgcaa attcctccgg 11580
   aaacagggac gagccccttt tttgcttttc ccagatgcat ccggtgctgc ggcagatgcg 11640
   cccccctcct cagcagcggc aagagcaaga gcagcggcag acatgcaggg caccctcccc 11700
   tcctcctacc gcgtcaggag gggcgacatc cgcggttgac gcggcagcag atggtgatta 11760
   cgaacccccg cggcgccggg cccggcacta cctggacttg gaggagggcg agggcctggc 11820
   gcggctagga gcgccctctc ctgagcggta cccaagggtg cagctgaagc gtgatacgcg 11880
   tgaggcgtac gtgccgcggc agaacctgtt tcgcgaccgc gagggagagg agcccgagga 11940
   gatgcgggat cgaaagttcc acgcagggcg cgagctgcgg catggcctga atcgcgagcg 12000
   gttgctgcgc gaggaggact ttgagcccga cgcgcgaacc gggattagtc ccgcgcgcgc 12060
   acacgtggcg gccgccgacc tggtaaccgc atacgagcag acggtgaacc aggagattaa 12120
   ctttcaaaaa agctttaaca accacgtgcg tacgcttgtg gcgcgcgagg aggtggctat 12180
   aggactgatg catctgtggg actttgtaag cgcgctggag caaaacccaa atagcaagcc 12240
   gctcatggcg cagctgttcc ttatagtgca gcacagcagg gacaacgagg cattcaggga 12300
   tgcgctgcta aacatagtag agcccgaggg ccgctggctg ctcgatttga taaacatcct 12360
   gcagagcata gtggtgcagg agcgcagctt gagcctggct gacaaggtgg ccgccatcaa 12420
   ctattccatg cttagcctgg gcaagtttta cgcccgcaag atataccata ccccttacgt 12480
   tcccatagac aaggaggtaa agatcgaggg gttctacatg cgcatggcgc tgaaggtgct 12540
   taccttgagc gacgacctgg gcgtttatcg caacgagcgc atccacaagg ccgtgagcgt 12600
   gagccggcgg cgcgagctca gcgaccgcga gctgatgcac agcctgcaaa gggccctggc 12660
   tggcacgggc agcggcgata gagaggccga gtcctacttt gacgcgggcg ctgacctgcg 12720
   ctgggcccca agccgacgcg ccctggaggc agctggggcc ggacctgggc tggcggtggc 12780
   acccgcgcgc gctggcaacg tcggcggcgt ggaggaatat gacgaggacg atgagtacga 12840
   gccagaggac ggcgagtact aagcggtgat gtttctgatc agatgatgca agacgcaacg 12900
   gacccggcgg tgcgggcggc gctgcagagc cagccgtccg gccttaactc cacggacgac 12960
   tggcgccagg tcatggaccg catcatgtcg ctgactgcgc gcaatcctga cgcgttccgg 13020
   cagcagccgc aggccaaccg gctctccgca attctggaag cggtggtccc ggcgcgcgca 13080
   aaccccacgc acgagaaggt gctggcgatc gtaaacgcgc tggccgaaaa cagggccatc 13140
   cggcccgacg aggccggcct ggtctacgac gcgctgcttc agcgcgtggc tcgttacaac 13200
   agcggcaacg tgcagaccaa cctggaccgg ctggtggggg atgtgcgcga ggccgtggcg 13260
   cagcgtgagc gcgcgcagca gcagggcaac ctgggctcca tggttgcact aaacgccttc 13320
   ctgagtacac agcccgccaa cgtgccgcgg ggacaggagg actacaccaa ctttgtgagc 13380
   gcactgcggc taatggtgac tgagacaccg caaagtgagg tgtaccagtc tgggccagac 13440
   tattttttcc agaccagtag acaaggcctg cagaccgtaa acctgagcca ggctttcaaa 13500
   aacttgcagg ggctgtgggg ggtgcgggct cccacaggcg accgcgcgac cgtgtctagc 13560
   ttgctgacgc ccaactcgcg cctgttgctg ctgctaatag cgcccttcac ggacagtggc 13620
   agcgtgtccc gggacacata cctaggtcac ttgctgacac tgtaccgcga ggccataggt 13680
   caggcgcatg tggacgagca tactttccag gagattacaa gtgtcagccg cgcgctgggg 13740
   caggaggaca cgggcagcct ggaggcaacc ctaaactacc tgctgaccaa ccggcggcag 13800
   aagatcccct cgttgcacag tttaaacagc gaggaggagc gcattttgcg ctacgtgcag 13860
   cagagcgtga gccttaacct gatgcgcgac ggggtaacgc ccagcgtggc gctggacatg 13920
   accgcgcgca acatggaacc gggcatgtat gcctcaaacc ggccgtttat caaccgccta 13980
   atggactact tgcatcgcgc ggccgccgtg aaccccgagt atttcaccaa tgccatcttg 14040
   aacccgcact ggctaccgcc ccctggtttc tacaccgggg gattcgaggt gcccgagggt 14100
   aacgatggat tcctctggga cgacatagac gacagcgtgt tttccccgca accgcagacc 14160
   ctgctagagt tgcaacagcg cgagcaggca gaggcggcgc tgcgaaagga aagcttccgc 14220
   aggccaagca gcttgtccga tctaggcgct gcggccccgc ggtcagatgc tagtagccca 14280
   tttccaagct tgatagggtc tcttaccagc actcgcacca cccgcccgcg cctgctgggc 14340
   gaggaggagt acctaaacaa ctcgctgctg cagccgcagc gcgaaaaaaa cctgcctccg 14400
   gcatttccca acaacgggat agagagccta gtggacaaga tgagtagatg gaagacgtac 14460
   gcgcaggagc acagggacgt gccaggcccg cgcccgccca cccgtcgtca aaggcacgac 14520
   cgtcagcggg gtctggtgtg ggaggacgat gactcggcag acgacagcag cgtcctggat 14580
   ttgggaggga gtggcaaccc gtttgcgcac cttcgcccca ggctggggag aatgttttaa 14640
   aaaaaaaaaa gcatgatgca aaataaaaaa ctcaccaagg ccatggcacc gagcgttggt 14700
   tttcttgtat tccccttagt atgcggcgcg cggcgatgta tgaggaaggt cctcctccct 14760
   cctacgagag tgtggtgagc gcggcgccag tggcggcggc gctgggttct cccttcgatg 14820
   ctcccctgga cccgccgttt gtgcctccgc ggtacctgcg gcctaccggg gggagaaaca 14880
   gcatccgtta ctctgagttg gcacccctat tcgacaccac ccgtgtgtac ctggtggaca 14940
   acaagtcaac ggatgtggca tccctgaact accagaacga ccacagcaac tttctgacca 15000
   cggtcattca aaacaatgac tacagcccgg gggaggcaag cacacagacc atcaatcttg 15060
   acgaccggtc gcactggggc ggcgacctga aaaccatcct gcataccaac atgccaaatg 15120
   tgaacgagtt catgtttacc aataagttta aggcgcgggt gatggtgtcg cgcttgccta 15180
   ctaaggacaa tcaggtggag ctgaaatacg agtgggtgga gttcacgctg cccgagggca 15240
   actactccga gaccatgacc atagacctta tgaacaacgc gatcgtggag cactacttga 15300
   aagtgggcag acagaacggg gttctggaaa gcgacatcgg ggtaaagttt gacacccgca 15360
   acttcagact ggggtttgac cccgtcactg gtcttgtcat gcctggggta tatacaaacg 15420
   aagccttcca tccagacatc attttgctgc caggatgcgg ggtggacttc acccacagcc 15480
   gcctgagcaa cttgttgggc atccgcaagc ggcaaccctt ccaggagggc tttaggatca 15540
   cctacgatga tctggagggt ggtaacattc ccgcactgtt ggatgtggac gcctaccagg 15600
   cgagcttgaa agatgacacc gaacagggcg ggggtggcgc aggcggcagc aacagcagtg 15660
   gcagcggcgc ggaagagaac tccaacgcgg cagccgcggc aatgcagccg gtggaggaca 15720
   tgaacgatca tgccattcgc ggcgacacct ttgccacacg ggctgaggag aagcgcgctg 15780
   aggccgaagc agcggccgaa gctgccgccc ccgctgcgca acccgaggtc gagaagcctc 15840
   agaagaaacc ggtgatcaaa cccctgacag aggacagcaa gaaacgcagt tacaacctaa 15900
   taagcaatga cagcaccttc acccagtacc gcagctggta ccttgcatac aactacggcg 15960
   accctcagac cggaatccgc tcatggaccc tgctttgcac tcctgacgta acctgcggct 16020
   cggagcaggt ctactggtcg ttgccagaca tgatgcaaga ccccgtgacc ttccgctcca 16080
   cgcgccagat cagcaacttt ccggtggtgg gcgccgagct gttgcccgtg cactccaaga 16140
   gcttctacaa cgaccaggcc gtctactccc aactcatccg ccagtttacc tctctgaccc 16200
   acgtgttcaa tcgctttccc gagaaccaga ttttggcgcg cccgccagcc cccaccatca 16260
   ccaccgtcag tgaaaacgtt cctgctctca cagatcacgg gacgctaccg ctgcgcaaca 16320
   gcatcggagg agtccagcga gtgaccatta ctgacgccag acgccgcacc tgcccctacg 16380
   tttacaaggc cctgggcata gtctcgccgc gcgtcctatc gagccgcact ttttgagcaa 16440
   gcatgtccat ccttatatcg cccagcaata acacaggctg gggcctgcgc ttcccaagca 16500
   agatgtttgg cggggccaag aagcgctccg accaacaccc agtgcgcgtg cgcgggcact 16560
   accgcgcgcc ctggggcgcg cacaaacgcg gccgcactgg gcgcaccacc gtcgatgacg 16620
   ccatcgacgc ggtggtggag gaggcgcgca actacacgcc cacgccgcca ccagtgtcca 16680
   cagtggacgc ggccattcag accgtggtgc gcggagcccg gcgctatgct aaaatgaaga 16740
   gacggcggag gcgcgtagca cgtcgccacc gccgccgacc cggcactgcc gcccaacgcg 16800
   cggcggcggc cctgcttaac cgcgcacgtc gcaccggccg acgggcggcc atgcgggccg 16860
   ctcgaaggct ggccgcgggt attgtcactg tgccccccag gtccaggcga cgagcggccg 16920
   ccgcagcagc cgcggccatt agtgctatga ctcagggtcg caggggcaac gtgtattggg 16980
   tgcgcgactc ggttagcggc ctgcgcgtgc ccgtgcgcac ccgccccccg cgcaactaga 17040
   ttgcaagaaa aaactactta gactcgtact gttgtatgta tccagcggcg gcggcgcgca 17100
   acgaagctat gtccaagcgc aaaatcaaag aagagatgct ccaggtcatc gcgccggaga 17160
   tctatggccc cccgaagaag gaagagcagg attacaagcc ccgaaagcta aagcgggtca 17220
   aaaagaaaaa gaaagatgat gatgatgaac ttgacgacga ggtggaactg ctgcacgcta 17280
   ccgcgcccag gcgacgggta cagtggaaag gtcgacgcgt aaaacgtgtt ttgcgacccg 17340
   gcaccaccgt agtctttacg cccggtgagc gctccacccg cacctacaag cgcgtgtatg 17400
   atgaggtgta cggcgacgag gacctgcttg agcaggccaa cgagcgcctc ggggagtttg 17460
   cctacggaaa gcggcataag gacatgctgg cgttgccgct ggacgagggc aacccaacac 17520
   ctagcctaaa gcccgtaaca ctgcagcagg tgctgcccgc gcttgcaccg tccgaagaaa 17580
   agcgcggcct aaagcgcgag tctggtgact tggcacccac cgtgcagctg atggtaccca 17640
   agcgccagcg actggaagat gtcttggaaa aaatgaccgt ggaacctggg ctggagcccg 17700
   aggtccgcgt gcggccaatc aagcaggtgg cgccgggact gggcgtgcag accgtggacg 17760
   ttcagatacc cactaccagt agcaccagta ttgccaccgc cacagagggc atggagacac 17820
   aaacgtcccc ggttgcctca gcggtggcgg atgccgcggt gcaggcggtc gctgcggccg 17880
   cgtccaagac ctctacggag gtgcaaacgg acccgtggat gtttcgcgtt tcagcccccc 17940
   ggcgcccgcg cggttcgagg aagtacggcg ccgccagcgc gctactgccc gaatatgccc 18000
   tacatccttc cattgcgcct acccccggct atcgtggcta cacctaccgc cccagaagac 18060
   gagcaactac ccgacgccga accaccactg gaacccgccg ccgccgtcgc cgtcgccagc 18120
   ccgtgctggc cccgatttcc gtgcgcaggg tggctcgcga aggaggcagg accctggtgc 18180
   tgccaacagc gcgctaccac cccagcatcg tttaaaagcc ggtctttgtg gttcttgcag 18240
   atatggccct cacctgccgc ctccgtttcc cggtgccggg attccgagga agaatgcacc 18300
   gtaggagggg catggccggc cacggcctga cgggcggcat gcgtcgtgcg caccaccggc 18360
   ggcggcgcgc gtcgcaccgt cgcatgcgcg gcggtatcct gcccctcctt attccactga 18420
   tcgccgcggc gattggcgcc gtgcccggaa ttgcatccgt ggccttgcag gcgcagagac 18480
   actgattaaa aacaagttgc atgtggaaaa atcaaaataa aaagtctgga ctctcacgct 18540
   cgcttggtcc tgtaactatt ttgtagaatg gaagacatca actttgcgtc tctggccccg 18600
   cgacacggct cgcgcccgtt catgggaaac tggcaagata tcggcaccag caatatgagc 18660
   ggtggcgcct tcagctgggg ctcgctgtgg agcggcatta aaaatttcgg ttccaccgtt 18720
   aagaactatg gcagcaaggc ctggaacagc agcacaggcc agatgctgag ggataagttg 18780
   aaagagcaaa atttccaaca aaaggtggta gatggcctgg cctctggcat tagcggggtg 18840
   gtggacctgg ccaaccaggc agtgcaaaat aagattaaca gtaagcttga tccccgccct 18900
   cccgtagagg agcctccacc ggccgtggag acagtgtctc cagaggggcg tggcgaaaag 18960
   cgtccgcgcc ccgacaggga agaaactctg gtgacgcaaa tagacgagcc tccctcgtac 19020
   gaggaggcac taaagcaagg cctgcccacc acccgtccca tcgcgcccat ggctaccgga 19080
   gtgctgggcc agcacacacc cgtaacgctg gacctgcctc cccccgccga cacccagcag 19140
   aaacctgtgc tgccaggccc gaccgccgtt gttgtaaccc gtcctagccg cgcgtccctg 19200
   cgccgcgccg ccagcggtcc gcgatcgttg cggcccgtag ccagtggcaa ctggcaaagc 19260
   acactgaaca gcatcgtggg tctgggggtg caatccctga agcgccgacg atgcttctga 19320
   atagctaacg tgtcgtatgt gtgtcatgta tgcgtccatg tcgccgccag aggagctgct 19380
   gagccgccgc gcgcccgctt tccaagatgg ctaccccttc gatgatgccg cagtggtctt 19440
   acatgcacat ctcgggccag gacgcctcgg agtacctgag ccccgggctg gtgcagtttg 19500
   cccgcgccac cgagacgtac ttcagcctga ataacaagtt tagaaacccc acggtggcgc 19560
   ctacgcacga cgtgaccaca gaccggtccc agcgtttgac gctgcggttc atccctgtgg 19620
   accgtgagga tactgcgtac tcgtacaagg cgcggttcac cctagctgtg ggtgataacc 19680
   gtgtgctgga catggcttcc acgtactttg acatccgcgg cgtgctggac aggggcccta 19740
   cttttaagcc ctactctggc actgcctaca acgccctggc tcccaagggt gccccaaatc 19800
   cttgcgaatg ggatgaagct gctactgctc ttgaaataaa cctagaagaa gaggacgatg 19860
   acaacgaaga cgaagtagac gagcaagctg agcagcaaaa aactcacgta tttgggcagg 19920
   cgccttattc tggtataaat attacaaagg agggtattca aataggtgtc gaaggtcaaa 19980
   cacctaaata tgccgataaa acatttcaac ctgaacctca aataggagaa tctcagtggt 20040
   acgaaactga aattaatcat gcagctggga gagtccttaa aaagactacc ccaatgaaac 20100
   catgttacgg ttcatatgca aaacccacaa atgaaaatgg agggcaaggc attcttgtaa 20160
   agcaacaaaa tggaaagcta gaaagtcaag tggaaatgca atttttctca actactgagg 20220
   cgaccgcagg caatggtgat aacttgactc ctaaagtggt attgtacagt gaagatgtag 20280
   atatagaaac cccagacact catatttctt acatgcccac tattaaggaa ggtaactcac 20340
   gagaactaat gggccaacaa tctatgccca acaggcctaa ttacattgct tttagggaca 20400
   attttattgg tctaatgtat tacaacagca cgggtaatat gggtgttctg gcgggccaag 20460
   catcgcagtt gaatgctgtt gtagatttgc aagacagaaa cacagagctt tcataccagc 20520
   ttttgcttga ttccattggt gatagaacca ggtacttttc tatgtggaat caggctgttg 20580
   acagctatga tccagatgtt agaattattg aaaatcatgg aactgaagat gaacttccaa 20640
   attactgctt tccactggga ggtgtgatta atacagagac tcttaccaag gtaaaaccta 20700
   aaacaggtca ggaaaatgga tgggaaaaag atgctacaga attttcagat aaaaatgaaa 20760
   taagagttgg aaataatttt gccatggaaa tcaatctaaa tgccaacctg tggagaaatt 20820
   tcctgtactc caacatagcg ctgtatttgc ccgacaagct aaagtacagt ccttccaacg 20880
   taaaaatttc tgataaccca aacacctacg actacatgaa caagcgagtg gtggctcccg 20940
   ggttagtgga ctgctacatt aaccttggag cacgctggtc ccttgactat atggacaacg 21000
   tcaacccatt taaccaccac cgcaatgctg gcctgcgcta ccgctcaatg ttgctgggca 21060
   atggtcgcta tgtgcccttc cacatccagg tgcctcagaa gttctttgcc attaaaaacc 21120
   tccttctcct gccgggctca tacacctacg agtggaactt caggaaggat gttaacatgg 21180
   ttctgcagag ctccctagga aatgacctaa gggttgacgg agccagcatt aagtttgata 21240
   gcatttgcct ttacgccacc ttcttcccca tggcccacaa caccgcctcc acgcttgagg 21300
   ccatgcttag aaacgacacc aacgaccagt cctttaacga ctatctctcc gccgccaaca 21360
   tgctctaccc tatacccgcc aacgctacca acgtgcccat atccatcccc tcccgcaact 21420
   gggcggcttt ccgcggctgg gccttcacgc gccttaagac taaggaaacc ccatcactgg 21480
   gctcgggcta cgacccttat tacacctact ctggctctat accctaccta gatggaacct 21540
   tttacctcaa ccacaccttt aagaaggtgg ccattacctt tgactcttct gtcagctggc 21600
   ctggcaatga ccgcctgctt acccccaacg agtttgaaat taagcgctca gttgacgggg 21660
   agggttacaa cgttgcccag tgtaacatga ccaaagactg gttcctggta caaatgctag 21720
   ctaactacaa cattggctac cagggcttct atatcccaga gagctacaag gaccgcatgt 21780
   actccttctt tagaaacttc cagcccatga gccgtcaggt ggtggatgat actaaataca 21840
   aggactacca acaggtgggc atcctacacc aacacaacaa ctctggattt gttggctacc 21900
   ttgcccccac catgcgcgaa ggacaggcct accctgctaa cttcccctat ccgcttatag 21960
   gcaagaccgc agttgacagc attacccaga aaaagtttct ttgcgatcgc accctttggc 22020
   gcatcccatt ctccagtaac tttatgtcca tgggcgcact cacagacctg ggccaaaacc 22080
   ttctctacgc caactccgcc cacgcgctag acatgacttt tgaggtggat cccatggacg 22140
   agcccaccct tctttatgtt ttgtttgaag tctttgacgt ggtccgtgtg caccggccgc 22200
   accgcggcgt catcgaaacc gtgtacctgc gcacgccctt ctcggccggc aacgccacaa 22260
   cataaagaag caagcaacat caacaacagc tgccgccatg ggctccagtg agcaggaact 22320
   gaaagccatt gtcaaagatc ttggttgtgg gccatatttt ttgggcacct atgacaagcg 22380
   ctttccaggc tttgtttctc cacacaagct cgcctgcgcc atagtcaata cggccggtcg 22440
   cgagactggg ggcgtacact ggatggcctt tgcctggaac ccgcactcaa aaacatgcta 22500
   cctctttgag ccctttggct tttctgacca gcgactcaag caggtttacc agtttgagta 22560
   cgagtcactc ctgcgccgta gcgccattgc ttcttccccc gaccgctgta taacgctgga 22620
   aaagtccacc caaagcgtac aggggcccaa ctcggccgcc tgtggactat tctgctgcat 22680
   gtttctccac gcctttgcca actggcccca aactcccatg gatcacaacc ccaccatgaa 22740
   ccttattacc ggggtaccca actccatgct caacagtccc caggtacagc ccaccctgcg 22800
   tcgcaaccag gaacagctct acagcttcct ggagcgccac tcgccctact tccgcagcca 22860
   cagtgcgcag attaggagcg ccacttcttt ttgtcacttg aaaaacatgt aaaaataatg 22920
   tactagagac actttcaata aaggcaaatg cttttatttg tacactctcg ggtgattatt 22980
   tacccccacc cttgccgtct gcgccgttta aaaatcaaag gggttctgcc gcgcatcgct 23040
   atgcgccact ggcagggaca cgttgcgata ctggtgttta gtgctccact taaactcagg 23100
   cacaaccatc cgcggcagct cggtgaagtt ttcactccac aggctgcgca ccatcaccaa 23160
   cgcgtttagc aggtcgggcg ccgatatctt gaagtcgcag ttggggcctc cgccctgcgc 23220
   gcgcgagttg cgatacacag ggttgcagca ctggaacact atcagcgccg ggtggtgcac 23280
   gctggccagc acgctcttgt cggagatcag atccgcgtcc aggtcctccg cgttgctcag 23340
   ggcgaacgga gtcaactttg gtagctgcct tcccaaaaag ggcgcgtgcc caggctttga 23400
   gttgcactcg caccgtagtg gcatcaaaag gtgaccgtgc ccggtctggg cgttaggata 23460
   cagcgcctgc ataaaagcct tgatctgctt aaaagccacc tgagcctttg cgccttcaga 23520
   gaagaacatg ccgcaagact tgccggaaaa ctgattggcc ggacaggccg cgtcgtgcac 23580
   gcagcacctt gcgtcggtgt tggagatctg caccacattt cggccccacc ggttcttcac 23640
   gatcttggcc ttgctagact gctccttcag cgcgcgctgc ccgttttcgc tcgtcacatc 23700
   catttcaatc acgtgctcct tatttatcat aatgcttccg tgtagacact taagctcgcc 23760
   ttcgatctca gcgcagcggt gcagccacaa cgcgcagccc gtgggctcgt gatgcttgta 23820
   ggtcacctct gcaaacgact gcaggtacgc ctgcaggaat cgccccatca tcgtcacaaa 23880
   ggtcttgttg ctggtgaagg tcagctgcaa cccgcggtgc tcctcgttca gccaggtctt 23940
   gcatacggcc gccagagctt ccacttggtc aggcagtagt ttgaagttcg cctttagatc 24000
   gttatccacg tggtacttgt ccatcagcgc gcgcgcagcc tccatgccct tctcccacgc 24060
   agacacgatc ggcacactca gcgggttcat caccgtaatt tcactttccg cttcgctggg 24120
   ctcttcctct tcctcttgcg tccgcatacc acgcgccact gggtcgtctt cattcagccg 24180
   ccgcactgtg cgcttacctc ctttgccatg cttgattagc accggtgggt tgctgaaacc 24240
   caccatttgt agcgccacat cttctctttc ttcctcgctg tccacgatta cctctggtga 24300
   tggcgggcgc tcgggcttgg gagaagggcg cttctttttc ttcttgggcg caatggccaa 24360
   atccgccgcc gaggtcgatg gccgcgggct gggtgtgcgc ggcaccagcg cgtcttgtga 24420
   tgagtcttcc tcgtcctcgg actcgatacg ccgcctcatc cgcttttttg ggggcgcccg 24480
   gggaggcggc ggcgacgggg acggggacga cacgtcctcc atggttgggg gacgtcgcgc 24540
   cgcaccgcgt ccgcgctcgg gggtggtttc gcgctgctcc tcttcccgac tggccatttc 24600
   cttctcctat aggcagaaaa agatcatgga gtcagtcgag aagaaggaca gcctaaccgc 24660
   cccctctgag ttcgccacca ccgcctccac cgatgccgcc aacgcgccta ccaccttccc 24720
   cgtcgaggca cccccgcttg aggaggagga agtgattatc gagcaggacc caggttttgt 24780
   aagcgaagac gacgaggacc gctcagtacc aacagaggat aaaaagcaag accaggacaa 24840
   cgcagaggca aacgaggaac aagtcgggcg gggggacgaa aggcatggcg actacctaga 24900
   tgtgggagac gacgtgctgt tgaagcatct gcagcgccag tgcgccatta tctgcgacgc 24960
   gttgcaagag cgcagcgatg tgcccctcgc catagcggat gtcagccttg cctacgaacg 25020
   ccacctattc tcaccgcgcg taccccccaa acgccaagaa aacggcacat gcgagcccaa 25080
   cccgcgcctc aacttctacc ccgtatttgc cgtgccagag gtgcttgcca cctatcacat 25140
   ctttttccaa aactgcaaga tacccctatc ctgccgtgcc aaccgcagcc gagcggacaa 25200
   gcagctggcc ttgcggcagg gcgctgtcat acctgatatc gcctcgctca acgaagtgcc 25260
   aaaaatcttt gagggtcttg gacgcgacga gaagcgcgcg gcaaacgctc tgcaacagga 25320
   aaacagcgaa aatgaaagtc actctggagt gttggtggaa ctcgagggtg acaacgcgcg 25380
   cctagccgta ctaaaacgca gcatcgaggt cacccacttt gcctacccgg cacttaacct 25440
   accccccaag gtcatgagca cagtcatgag tgagctgatc gtgcgccgtg cgcagcccct 25500
   ggagagggat gcaaatttgc aagaacaaac agaggagggc ctacccgcag ttggcgacga 25560
   gcagctagcg cgctggcttc aaacgcgcga gcctgccgac ttggaggagc gacgcaaact 25620
   aatgatggcc gcagtgctcg ttaccgtgga gcttgagtgc atgcagcggt tctttgctga 25680
   cccggagatg cagcgcaagc tagaggaaac attgcactac acctttcgac agggctacgt 25740
   acgccaggcc tgcaagatct ccaacgtgga gctctgcaac ctggtctcct accttggaat 25800
   tttgcacgaa aaccgccttg ggcaaaacgt gcttcattcc acgctcaagg gcgaggcgcg 25860
   ccgcgactac gtccgcgact gcgtttactt atttctatgc tacacctggc agacggccat 25920
   gggcgtttgg cagcagtgct tggaggagtg caacctcaag gagctgcaga aactgctaaa 25980
   gcaaaacttg aaggacctat ggacggcctt caacgagcgc tccgtggccg cgcacctggc 26040
   ggacatcatt ttccccgaac gcctgcttaa aaccctgcaa cagggtctgc cagacttcac 26100
   cagtcaaagc atgttgcaga actttaggaa ctttatccta gagcgctcag gaatcttgcc 26160
   cgccacctgc tgtgcacttc ctagcgactt tgtgcccatt aagtaccgcg aatgccctcc 26220
   gccgctttgg ggccactgct accttctgca gctagccaac taccttgcct accactctga 26280
   cataatggaa gacgtgagcg gtgacggtct actggagtgt cactgtcgct gcaacctatg 26340
   caccccgcac cgctccctgg tttgcaattc gcagctgctt aacgaaagtc aaattatcgg 26400
   tacctttgag ctgcagggtc cctcgcctga cgaaaagtcc gcggctccgg ggttgaaact 26460
   cactccgggg ctgtggacgt cggcttacct tcgcaaattt gtacctgagg actaccacgc 26520
   ccacgagatt aggttctacg aagaccaatc ccgcccgcca aatgcggagc ttaccgcctg 26580
   cgtcattacc cagggccaca ttcttggcca attgcaagcc atcaacaaag cccgccaaga 26640
   gtttctgcta cgaaagggac ggggggttta cttggacccc cagtccggcg aggagctcaa 26700
   cccaatcccc ccgccgccgc agccctatca gcagcagccg cgggcccttg cttcccagga 26760
   tggcacccaa aaagaagctg cagctgccgc cgccacccac ggacgaggag gaatactggg 26820
   acagtcaggc agaggaggtt ttggacgagg aggaggagga catgatggaa gactgggaga 26880
   gcctagacga ggaagcttcc gaggtcgaag aggtgtcaga cgaaacaccg tcaccctcgg 26940
   tcgcattccc ctcgccggcg ccccagaaat cggcaaccgg ttccagcatg gctacaacct 27000
   ccgctcctca ggcgccgccg gcactgcccg ttcgccgacc caaccgtaga tgggacacca 27060
   ctggaaccag ggccggtaag tccaagcagc cgccgccgtt agcccaagag caacaacagc 27120
   gccaaggcta ccgctcatgg cgcgggcaca agaacgccat agttgcttgc ttgcaagact 27180
   gtgggggcaa catctccttc gcccgccgct ttcttctcta ccatcacggc gtggccttcc 27240
   cccgtaacat cctgcattac taccgtcatc tctacagccc atactgcacc ggcggcagcg 27300
   gcagcggcag caacagcagc ggccacacag aagcaaaggc gaccggatag caagactctg 27360
   acaaagccca agaaatccac agcggcggca gcagcaggag gaggagcgct gcgtctggcg 27420
   cccaacgaac ccgtatcgac ccgcgagctt agaaacagga tttttcccac tctgtatgct 27480
   atatttcaac agagcagggg ccaagaacaa gagctgaaaa taaaaaacag gtctctgcga 27540
   tccctcaccc gcagctgcct gtatcacaaa agcgaagatc agcttcggcg cacgctggaa 27600
   gacgcggagg ctctcttcag taaatactgc gcgctgactc ttaaggacta gtttcgcgcc 27660
   ctttctcaaa tttaagcgcg aaaactacgt catctccagc ggccacaccc ggcgccagca 27720
   cctgtcgtca gcgccattat gagcaaggaa attcccacgc cctacatgtg gagttaccag 27780
   ccacaaatgg gacttgcggc tggagctgcc caagactact caacccgaat aaactacatg 27840
   agcgcgggac cccacatgat atcccgggtc aacggaatcc gcgcccaccg aaaccgaatt 27900
   ctcttggaac aggcggctat taccaccaca cctcgtaata accttaatcc ccgtagttgg 27960
   cccgctgccc tggtgtacca ggaaagtccc gctcccacca ctgtggtact tcccagagac 28020
   gcccaggccg aagttcagat gactaactca ggggcgcagc ttgcgggcgg ctttcgtcac 28080
   agggtgcggt cgcccgggca gggtataact cacctgacaa tcagagggcg aggtattcag 28140
   ctcaacgacg agtcggtgag ctcctcgctt ggtctccgtc cggacgggac atttcagatc 28200
   ggcggcgccg gccgtccttc attcacgcct cgtcaggcaa tcctaactct gcagacctcg 28260
   tcctctgagc cgcgctctgg aggcattgga actctgcaat ttattgagga gtttgtgcca 28320
   tcggtctact ttaacccctt ctcgggacct cccggccact atccggatca atttattcct 28380
   aactttgacg cggtaaagga ctcggcggac ggctacgact gaatgttaag tggagaggca 28440
   gagcaactgc gcctgaaaca cctggtccac tgtcgccgcc acaagtgctt tgcccgcgac 28500
   tccggtgagt tttgctactt tgaattgccc gaggatcata tcgagggccc ggcgcacggc 28560
   gtccggctta ccgcccaggg agagcttgcc cgtagcctga ttcgggagtt tacccagcgc 28620
   cccctgctag ttgagcggga caggggaccc tgtgttctca ctgtgatttg caactgtcct 28680
   aaccttggat tacatcaaga tcctctagtt ataactagag tacccgggga tcttattccc 28740
   tttaactaat aaaaaaaaat aataaagcat cacttactta aaatcagtta gcaaatttct 28800
   gtccagttta ttcagcagca cctccttgcc ctcctcccag ctctggtatt gcagcttcct 28860
   cctggctgca aactttctcc acaatctaaa tggaatgtca gtttcctcct gttcctgtcc 28920
   atccgcaccc actatcttca tgttgttgca gatgaagcgc gcaagaccgt ctgaagatac 28980
   cttcaacccc gtgtatccat atgacacgga aaccggtcct ccaactgtgc cttttcttac 29040
   tcctcccttt gtatccccca atgggtttca agagagtccc cctggggtac tctctttgcg 29100
   cctatccgaa cctctagtta cctccaatgg catgcttgcg ctcaaaatgg gcaacggcct 29160
   ctctctggac gaggccggca accttacctc ccaaaatgta accactgtga gcccacctct 29220
   caaaaaaacc aagtcaaaca taaacctgga aatatctgca cccctcacag ttacctcaga 29280
   agccctaact gtggctgccg ccgcacctct aatggtcgcg ggcaacacac tcaccatgca 29340
   atcacaggcc ccgctaaccg tgcacgactc caaacttagc attgccaccc aaggacccct 29400
   cacagtgtca gaaggaaagc tagccctgca aacatcaggc cccctcacca ccaccgatag 29460
   cagtaccctt actatcactg cctcaccccc tctaactact gccactggta gcttgggcat 29520
   tgacttgaaa gagcccattt atacacaaaa tggaaaacta ggactaaagt acggggctcc 29580
   tttgcatgta acagacgacc taaacacttt gaccgtagca actggtccag gtgtgactat 29640
   taataatact tccttgcaaa ctaaagttac tggagccttg ggttttgatt cacaaggcaa 29700
   tatgcaactt aatgtagcag gaggactaag gattgattct caaaacagac gccttatact 29760
   tgatgttagt tatccgtttg atgctcaaaa ccaactaaat ctaagactag gacagggccc 29820
   tctttttata aactcagccc acaacttgga tattaactac aacaaaggcc tttacttgtt 29880
   tacagcttca aacaattcca aaaagcttga ggttaaccta agcactgcca aggggttgat 29940
   gtttgacgct acagccatag ccattaatgc aggagatggg cttgaatttg gttcacctaa 30000
   tgcaccaaac acaaatcccc tcaaaacaaa aattggccat ggcctagaat ttgattcaaa 30060
   caaggctatg gttcctaaac taggaactgg ccttagtttt gacagcacag gtgccattac 30120
   agtaggaaac aaaaataatg ataagctaac tttgtggacc acaccagctc catctcctaa 30180
   ctgtagacta aatgcagaga aagatgctaa actcactttg gtcttaacaa aatgtggcag 30240
   tcaaatactt gctacagttt cagttttggc tgttaaaggc agtttggctc caatatctgg 30300
   aacagttcaa agtgctcatc ttattataag atttgacgaa aatggagtgc tactaaacaa 30360
   ttccttcctg gacccagaat attggaactt tagaaatgga gatcttactg aaggcacagc 30420
   ctatacaaac gctgttggat ttatgcctaa cctatcagct tatccaaaat ctcacggtaa 30480
   aactgccaaa agtaacattg tcagtcaagt ttacttaaac ggagacaaaa ctaaacctgt 30540
   aacactaacc attacactaa acggtacaca ggaaacagga gacacaactc caagtgcata 30600
   ctctatgtca ttttcatggg actggtctgg ccacaactac attaatgaaa tatttgccac 30660
   atcctcttac actttttcat acattgccca agaataaaga atcgtttgtg ttatgtttca 30720
   acgtgtttat ttttcaattg cagaaaattt caagtcattt ttcattcagt agtatagccc 30780
   caccaccaca tagcttatac agatcaccgt accttaatca aactcacaga accctagtat 30840
   tcaacctgcc acctccctcc caacacacag agtacacagt cctttctccc cggctggcct 30900
   taaaaagcat catatcatgg gtaacagaca tattcttagg tgttatattc cacacggttt 30960
   cctgtcgagc caaacgctca tcaagtgata ttaataaact ccccgggcag ctcacttaag 31020
   ttcatgtcgc tgtccagctg ctgagccaca ggctgctgtc caacttgcgg ttgcttaacg 31080
   ggcggcgaag gagaagtcca cgcctacatg gggggagagt cataatcgtg catcaggata 31140
   gggcggtggt gctgcagcag cgcgcgaata aactgctgcc gccgccgctc cgtcctgcag 31200
   gaatacaaca tggcagtggt ctcctcagcg atgattcgca ccgcccgcag cataaggcgc 31260
   ttgtcctccg ggcacagcag cgcaccctga tctcacttaa atcagcacag taactgcagc 31320
   acagcaccac aatattgttc aaaatcccac agtgcaaggc gctgtatcca aagctcatgg 31380
   cggggaccac agaacccacg tggccatcat accacaagcg caggtagatt aagtggcgac 31440
   ccctcataaa cacgctggac ataaacatta cctcttttgg catgttgtaa ttcaccacct 31500
   cccggtacca tataaacctc tgattaaaca tggcgccatc caccaccatc ctaaaccagc 31560
   tggccaaaac ctgccccgcc gggntataca ctgcagggaa ccgggacttg gacaatgaca 31620
   agtgggagag cccaggactc gtaaccatgg atcatcatgc tcgtcatgat atcaatgttg 31680
   gcacaacaca ggcacacgtg catacacttc ctcaggatta caagctcctc ccgcgttaga 31740
   accatatccc agggaacaac ccattcctga atcagcgtaa atcccacact gcagggaaga 31800
   cctcgcacgt aactcacgtt gtgcattgtc aaagtgttac attcgggcag cagcggatga 31860
   tcctccagta tggtagcgcg ggtttctgtc tcaaaaggag gtagacgatc cctactgtac 31920
   ggagtgcgcc gagacaaccg agatcgtgtt ggtcgtagtg tcatgccaaa tggaacgccg 31980
   gacgtagtca tatttcctga agcaaaacca ggtgcgggcg tgacaaacag atctgcgtct 32040
   ccggtctcgc cgcttagatc gctctgtgta gtagttgtag tatatccact ctctcaaagc 32100
   atccaggcgc cccctggctt cgggttctat gtaaactcct tcatgcgccg ctgccctgat 32160
   aacatccacc accgcagaat aagccacacc cagccaacct acacattcgt tctgcgagtc 32220
   acacacggga ggagcgggaa gagctggaag aaccatgttt ttttttttat tccaaaagat 32280
   tatccaaaac ctcaaaatga agatctatta agtgaacgcg ctcccctccg gtggcgtggt 32340
   caaactctac agccaaagaa cagataatgg catttgtaag atgttgcaca atggcttcca 32400
   aaaggcaaac ggccctcacg tccaagtgga cgtaaaggct aaacccttca gggtgaatct 32460
   cctctataaa cattccagca ccttcaacca tgcccaaata attctcatct cgccaccttc 32520
   tcaatatatc tctaagcaaa tcccgaatat ttaagtccgg gccattgtaa aaaatttggc 32580
   tccagagcgc cctccacctt cagcctcaag cagcgaatca tgattgcaaa aattcaggtt 32640
   cctcacagac ctgtataaga ttcaaaagcg gaacattaac aaaaataccg cgatcccgta 32700
   ggtcccttcg cagggccagc tgaacataat cgtgcaggtc tgcacggacc agcgcggcca 32760
   cttccccgcc aggaaccatg acaaaagaac ccacactgat tatgacacgc atactcggag 32820
   ctatgctaac cagcgtagcc ccgatgtaag cttgttgcat gggcggcgat ataaaatgca 32880
   aggtgctgct caaaaaatca ggcaaagcct cgcgcaaaaa agaaagcaca tcgtagtcat 32940
   gctcatgcag ataaaggcag gtaagctccg gaaccaccac agaaaaagac accatttttc 33000
   tctcaaacat gtctgcgggt ttctgcataa acacaaaata aaataacaaa aaaacattta 33060
   aacattagaa gcctgtctta caacaggaaa aacaaccctt ataagcataa gacggactac 33120
   ggccatgccg gcgtgaccgt aaaaaaactg gtcaccgtga ttaaaaagca ccaccgacag 33180
   ctcctcggtc atgtccggag tcataatgta agactcggta aacacatcag gttgattcat 33240
   cggtcagtgc taaaaagcga ccgaaatagc ccgggggaat acatacccgc aggcgtagag 33300
   acaacattac agcccccata ggaggtataa caaaattaat aggagagaaa aacacataaa 33360
   cacctgaaaa accctcctgc ctaggcaaaa tagcaccctc ccgctccaga acaacataca 33420
   gcgcttcaca gcggcagcct aacagtcagc cttaccagta aaaaagaaaa cctattaaaa 33480
   aaacaccact cgacacggca ccagctcaat cagtcacagt gtaaaaaagg gccaagtgca 33540
   gagcgagtat atataggact aaaaaatgac gtaacggtta aagtccacaa aaaacaccca 33600
   gaaaaccgca cgcgaaccta cgcccagaaa cgaaagccaa aaaacccaca acttcctcaa 33660
   atcgtcactt ccgttttccc acgttacgta acttcccatt ttaagaaaac tacaattccc 33720
   aacacataca agttactccg ccctaaaacc tacgtcaccc gccccgttcc cacgccccgc 33780
   gccacgtcac aaactccacc ccctcattat catattggct tcaatccaaa ataaggtata 33840
   ttattgatga tgtta 33855